# EUROPEAN PATENT SPECIFICATION

(11) **EP 3 993 888 B1**
(45) Date of publication and mention of the grant of the patent: **22.11.2023**
(21) Application number: 20739578.1
(22) Date of filing: 01.07.2020
(51) Int. Cl.: B01D 15/36, B01J 41/07, B01J 41/13, B01J 41/20

(54) **ANTIBODY DRUG CONJUGATE PURIFICATION**
REINIGUNG VON ANTIKÖRPER-ARZNEIMITTEL-KONJUGATEN
PURIFICATION D'UN CONJUGUÉ ANTICORPS-MÉDICAMENT

(30) Priority: 03.07.2019 EP 19184130
(43) Date of publication of application: 11.05.2022
(73) Proprietor: Merck Patent GmbH, 64293 Darmstadt (DE)
(72) Inventor: SKUDAS, Romas, 55116 MAINZ (DE); HOLZGREVE, Annika, 63500 SELIGENSTADT (DE); SCHULTE, Michael, 64293 Darmstadt (DE); PEECK, Lars, 64293 Darmstadt (DE); ZORN, Dominic, 64293 Darmstadt (DE)
(86) International application number: PCT/EP2020/068437
(87) International publication number: WO 2021/001387

(56) References cited:
- EP-A2- 0 129 719
- WO-A1-95/13861
- WO-A1-2007/014591
- WO-A1-2017/109619
- CN-B- 104 208 719

## Description

The present invention relates to an ion exchange separation material with amino-acid based endgroups. This material is especially suitable for the separation and purification of ADCs.

Antibody drug conjugates (ADCs) are a new class of high potency drugs typically dedicated for cancer treatment. In these conjugates the antibody molecule is dedicated for the specific recognition of cancer cells, the toxic payload carried by the antibody molecule destroys them. Examples of commercially available ADCs are Adcetris^{®} and Kadcyla^{®}.

These new drugs are manufactured by conjugating a toxic molecule (payload) on the antibody molecule. This is typically achieved though controlled chemical conjugation methods with specific amino acid residues exposed on the surface of the antibody, for example: Lysine or Cystein after cysteine bond reduction. Both approaches result in an heterogeneous mixture of ADC species with variable drug to antibody ratio (DAR's). The pathway of lysine conjugation creates more than 100 possibilities and will lead to an heterogeneous population of different DAR's (0,1, 2, 3, 4 ...>10). The pathway of cysteine conjugation will lead to a population of even DAR's (e.g. 0, 2, 4, 6 and 8) utilizing the 4 disulfide bridges of the antibody. To minimize the heterogeneity of ADC species site specific conjugation methods can be used. These methods have better control on the exact number and location of payload molecules linked to the antibodies, but is challenging due to the limited amount of toxic molecules conjugated (e.g. usually only 2) and limited choices of conjugation sites, as it may affect the efficacy of the drug. To overcome the manufacturing challenges in the ADC heterogeneity, some chromatographic technologies can be used to remove undesired low DAR and high DAR species. Low DAR species are undesired, as they are competing with the antibody drug conjugate and reduce the efficacy of the drug. The high toxic payload carrying DAR species (> 6), are also undesired, as they have effect on the drug efficiency and lifetime (K.J. Hamblett et al. in Clinical Cancer Research 10 (2004) 7063-7070). Further information about ADCs can be found in Rachel S. Zolot, Satarupa Basu, Ryan P. Million. Antibody-drug conjugates. Nature reviews Drug Discovery. 2013, 12: 259-260. Details about exemplary ADCs can be found in WO 2011/139985, especially in Example 8.

Hydrophobic interaction chromatography is sometimes applied for the purification of ADC species post conjugation process (US15104359). Unfortunately this method is limited due to low ADC stability in high salt concentration solutions and low binding capacity (~10 mg/ml) causing high purification costs and low purification efficiency. Therefore, there is an increasing need in the industry to enhance the efficiency of the ADC drugs obtaining mainly DAR2 and DAR4 species and enabling the best pharmaco-kinetics, efficacy, half-life, and tolerability.

Consequently it would be favourable to find a way to provide for a robust and reliable antibody drug conjugate purification step, applicable effectively in a wide range of conditions, where different DAR species are efficiently separated. This would also be beneficial for reducing the purification costs.

It has been found that an ion exchange material carrying covalently attached leucine residues or similar residues can be used for the purification of ADC's enabling an efficient DAR (drug antibody ratio) species separation at >10 mg ADC/ml material capacities, where in a more preferred embodiment the capacity is between 10-80 mg/ml. Moreover, this innovative ion exchange material can be used in high conductivity >10 mS/cm, where in more preferred embodiment the conductivity is between 10 - 40 mS/cm. Surprisingly, it was possible to separate each individual DAR species, regardless their conjugation procedure using a solvent pH gradient. Moreover, the application of this innovative ion exchange material showed significant economic advantages compared to hydrophobic interaction or hydroxy apatite materials, assuring >90% product recovery rates.

The present invention is therefore directed to separation materials for ion exchange chromatography based on a hydroxyl-containing base matrix, to the surfaces of which polymer chains are grafted by covalent bonding, characterised in that
a) the base support contains hydroxyl groups,
b) the polymer chains are covalently bonded to the support,
c) the polymer chains comprise amino acid end groups -N(Y)-R3 with Y being independently from each other H or CH3, preferably H, and R3 being -CHCOOMR4

with R4 being C1 to C4 alkyl, like methyl, ethyl, propyl, isopropyl, butyl, isobutyl, preferably isopropyl and isobutyl, very preferred isobutyl, or C1 to C4 perfluoroalkyl
and M being H, Na, K, or NH4+.

Preferrably, the monomer units of the polymer chains are linked in a linear manner and each monomer unit comprises an end group -N(Y)-R3.

Preferrably, the ionic density is between 10-1200 µeq/g.

In a preferred embodiment, Y is H and R4 is isopropyl and/or isobutyl.

In a preferred embodiment, the hydroxylgroup containing base matrix comprises aliphatic hydroxyl groups.

In a preferred embodiment, the base matrix is a copolymer formed by copolymerisation of at least one compound from the group a) and b) with
a) at least one hydrophilically substituted alkyl vinyl ether of the formula I 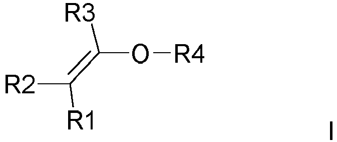
   where R1, R2, R3, independently of one another, can be H or C1 to C6 alkyl, preferably H or -CH₃,
   and R4 is a radical which carries at least one hydroxyl group
      and
b) at least one crosslinking agent conforming to formula II and/or III and/or IV with 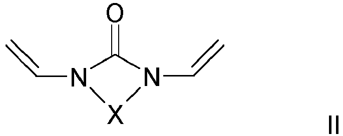 where X is a divalent alkyl radical having 2 to 5 C atoms, preferably 2 or 3 C atoms, in which one or more methylene groups which are not adjacent and are not located in the direct vicinity of N may be replaced by O, C=O, S, S=O, SO₂, NH, NOH or N and one or more H atoms of the methylene groups may be substituted, independently of one another, by hydroxyl groups, C1-C6-alkyl, halogen, NH₂, C5-C10-aryl, NH-(C1-C8)-alkyl, N-(C1-C8)-alkyl_{2,} C1-C6-alkoxy or C1-C6-alkyl-OH, and 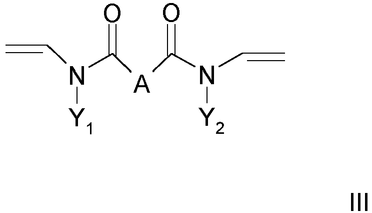 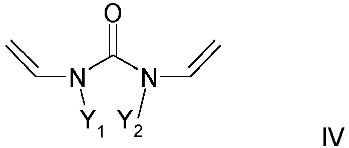
   where Y1 and Y2 in formula III and IV are, independently of one another,
   C1 to C10 alkyl or cycloalkyl, where one or more non-adjacent methylene groups or methylene groups which are not located in the direct vicinity of N may be replaced by O, C=O, S, S=O, SO₂, NH, NOH or N and one or more H of the methylene groups may be substituted, independently of one another, by hydroxyl groups, C1-C6-alkyl, halogen, NH₂, C5-C10-aryl, NH(C1-C8)alkyl, N(C1-C8)alkyl_{2,} C1-C6-alkoxy or C1-C6-alkyl-OH,
   or C6 to C18 aryl, where one or more H in the aryl system may be substituted, independently of one another, by hydroxyl groups, C1-C6-alkyl, halogen, NH₂, NH(C1-C8)alkyl, N(C1-C8)alkyl_{2,} C1-C6-alkoxy or C1-C6-alkyl-OH and
   A is a divalent alkyl radical having 2 to 5 C atoms, preferably 2 or 3 C atoms, in which one or more non-adjacent methylene groups or methylene groups which are not located in the direct vicinity of N may be replaced by O, C=O, S, S=O, SO₂, NH, NOH or N and one or more H of the methylene groups may be substituted, independently of one another, by hydroxyl groups, C1-C6-alkyl, halogen, NH₂, C5-C10-aryl, NH(C1-C8)alkyl, N(C1-C8)alkyl_{2,} C1-C6-alkoxy or C1-C6-alkyl-OH.

R4 in formula I is typically an alkyl radical, a cycloaliphatic radical or an aryl radical which carries at least one hydroxyl group.

In a very preferred embodiment the matrix is formed by copolymerisation of a hydrophilically substituted alkyl vinyl ether employed selected from the group of 1,4-butanediol monovinyl ether, 1,5-pentanediol monovinyl ether, diethylene glycol monovinyl ether or cyclohexanedimethanol monovinyl ether and divinylethyleneurea (1,3-divinylimidazolin-2-one) as crosslinking agent.

Especially preferred appropriate matrixes are disclosed for instance in WO 2007/014591 A1.

In a preferred embodiment, the separation material is preparable by subjecting the hydroxyl group containing base matrix to a cerium (IV) catalyzed graft polymerization of monomers according to formula V 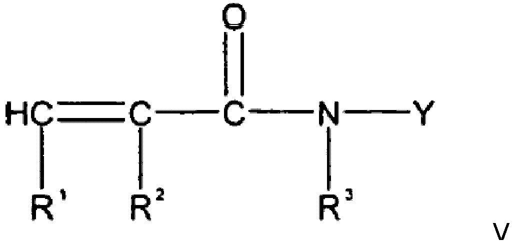
with R¹, R² and Y being independently from each other H or CH₃, preferably H,
R³ being -CHCOOMR⁴
with R⁴ being C1 to C4 alkyl, like methyl, ethyl, propyl, isopropyl, butyl, isobutyl, preferably isopropyl and isobutyl, very preferred isobutyl, or C1 to C4 perfluoroalkyl
and M being H, Na, K, or NH₄⁺.

This graft polymerization is preferably performed according to US 5453186 page 9 example 8.

The present invention is further directed to the chromatographic purification and/or separation of antibody drug conjugates (ADCs) by contacting the sample comprising the ADCs with a separation material comprising of a hydroxyl group containing base matrix, to the surfaces of which polymer chains are grafted by covalent bonding, characterised in that
a) the base support contains hydroxyl groups,
b) the polymers are covalently bonded to the support via the hydroxyl groups,
c) the polymers comprise end groups -N(Y)-R3 with

Y being independently from each other H or CH3, preferably H, and
R3 being -CHCOOMR4
with R4 being C1 to C4 alkyl, like methyl, ethyl, propyl, isopropyl, butyl, isobutyl, preferably isopropyl and isobutyl, very preferred isobutyl, or C1 to C4 perfluoroalkyl
and M being H, Na, K, or NH₄⁺.

In a preferred embodiment, the ADC target molecules are bound to the separation matrix at a pH between 2 and 7, optionally washed and eluted by increasing the pH value to an alkaline pH, preferrably a value above 9, e.g. between 9 and 11, preferrably to around 10.

In a preferred embodiment, the sample is applied to the separation matrix at an ionic density between 10-1200 µeq/g.

In a preferred embodiment, between 10 mg and 100 mg ADCs are bound per ml of the separation material.
Figure 1 shows a schematic view of a separation material according to the present invention with the base material (dot) that is functionalized with a linear polymer built by polymerization of acryloylleucine monomers.
Figure 2 shows a schematic view of a separation material according to the present invention with the base material (dot) that is functionalized with a linear polymer built by polymerization of acryloylvaline monomers.
Figures 3 shows the reaction scheme for valine and leucine with acrylic acid chloride.
Figure 4 shows elution peak of the bound ADC on the separation matrix at 10 mg ADC/ ml CV loading and 250 mM NaCl. UV adsorption signal trace in solid line and pH signal trace in dashed line.

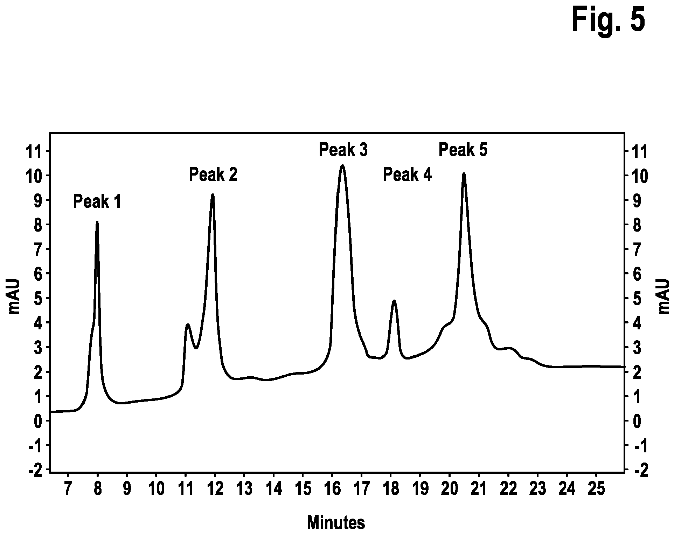

Figure 5 shows qualitative example of antibody drug conjugate species elution using linear gradient of decreasing conductivity and increasing 2-propanol quantity on an analytical HPLC column (mAbPac HIC-Butyl 5 µm, 4.6 × 100 mm (P/N: 088558) von Thermo Fisher Scientific). Buffer A consists of 1.5 M ammonium sulfate, 0,05 M Phosphate pH 7.0 and 5% 2-propanol, whereby buffer B consists of 0,05 M Phosphate pH 7.0 and 20% 2-propanol. HPLC column is operated in 1 ml/min flow rate and gradient from buffer A to buffer B is achieved in 20 minutes. ADC sample is diluted in buffer A at 5 mg/ml concentration and 10µl is injected on the HPLC column prior gradient elution.
Figure 6 shows analytical results of sample fraction characterization displayed in quantitative area of analyzed elution fractions (5A1 to 5B7), including loaded ADC (load) at 10 mg ADC/ ml CV loading and 250 mM NaCl.
Figure 7 shows elution peak of the bound ADC on the separation matrix at 30 mg ADC/ ml CV loading and 250 mM NaCl. UV adsorption signal trace in solid line and pH signal trace in dashed line.
Figure 8 represents analytical results of sample fraction characterization displayed in quantitative area of analyzed elution fractions (5A2 to 5C6), including loaded ADC (load) at 30 mg ADC/ ml CV loading and 250 mM NaCl.
Figure 9 represents deconvoluted mass spectrum for the Adcetris^{®} sample, showing a distribution of DAR species raging from the DAR0 to DAR4 species. DAR6 species are not detectable due to low ionization and method overload of lower DAR species.
Figure 10 shows deconvoluted mass spectrum for the elution fraction 5A7, representing the maximum of the first elution peak. The obtained molecular weight is for the DAR2 species, without obvious presence of other DAR species.
Figure 11 shows deconvoluted mass spectrum for the elution fraction 5B4, representing the maximum of the second elution peak. The obtained molecular weight is for the DAR4 species, without obvious presence of other DAR species.
Figure 12 shows deconvoluted mass spectrum for the elution fraction 5B11, representing the maximum of the third elution peak. The obtained molecular weight is for the DAR4 species and DAR6 species. The signal strength is not quantitative.
Figure 13 represents elution peak of the bound ADC on the separation matrix at 60 mg ADC/ ml CV loading and 250 mM NaCl. UV adsorption signal trace in solid line and pH signal trace in dashed line.
Figure 14 shows analytical results of sample fraction characterization displayed in quantitative area of analyzed elution fractions (1A1 to 3G12), including loaded ADC (load) at 60 mg ADC/ ml CV loading and 250 mM NaCl.
Figure 15 shows elution peak of the bound ADC at 30 mg ADC/ ml CV loading and 250 mM NaCl using Capto^{™} MMC ImpRes resin. UV adsorption signal trace in solid line and pH signal trace in dashed line.
Figure 16 represents analytical results of sample fraction characterization displayed in quantitative area of analyzed elution fractions (4A3 to 2C2), including loaded ADC (load) at 30 mg ADC/ ml CV loading and 250 mM NaCl.
Figure 17 shows elution peak of the bound ADC on the separation matrix at 10 mg ADC/ ml CV loading and 50 mM NaCl. UV adsorption signal trace in solid line and pH signal trace in dashed line.
Figure 18 shows elution peak of the bound ADC on the separation matrix at 10 mg ADC/ ml CV loading and 100 mM NaCl. UV adsorption signal trace in solid line and pH signal trace in dashed line.
Figure 19 shows elution peak of the bound ADC on the separation matrix at 10 mg ADC/ ml CV loading and 150 mM NaCl. UV adsorption signal trace in solid line and pH signal trace in dashed line.
Figure 20 shows elution peak of the bound ADC on the separation matrix at 10 mg ADC/ ml CV loading and 200 mM NaCl. UV adsorption signal trace in solid line and pH signal trace in dashed line.
Figure 21 shows elution peak of the bound ADC on the separation matrix at 10 mg ADC/ ml CV loading and 300 mM NaCl. UV adsorption signal trace in solid line and pH signal trace in dashed line.
Figure 22 shows elution peak of the bound ADC on the separation matrix at 10 mg ADC/ ml CV loading and 350mM NaCl. UV adsorption signal trace in solid line and pH signal trace in dashed line.
Figure 23 shows elution peak of the bound ADC on the separation matrix at 10 mg ADC/ ml CV loading and 300 mM NaCl using step approach. UV adsorption signal trace in solid line and pH signal trace in dashed line.
Figure 24 shows elution peak of the bound ADC on the separation matrix at 30 mg ADC/ ml CV loading and 250 mM NaCl. UV adsorption signal trace in solid line and pH signal trace in dashed line.

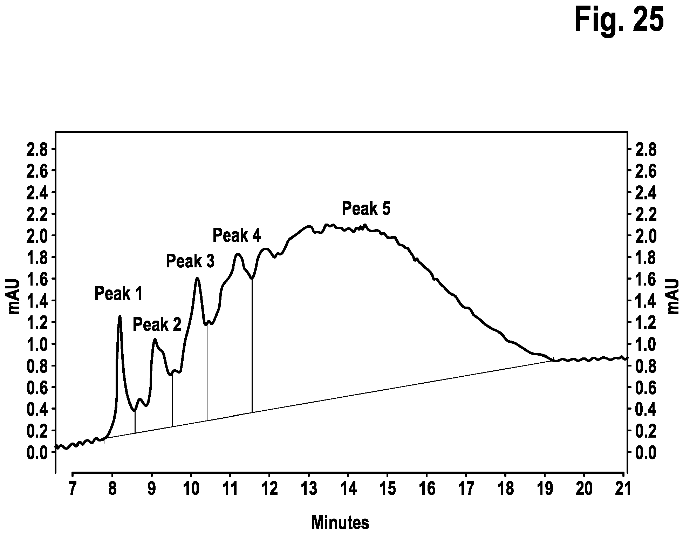

Figure 25 represents qualitative example of antibody drug conjugate species elution using linear gradient of decreasing conductivity and increasing 2-propanol quantity on an analytical HPLC column (mAbPac HIC-Butyl 5 µm, 4.6 × 100 mm (P/N: 088558) von Thermo Fisher Scientific). Buffer A consists of 1.5 M ammonium sulfate, 0,05 M Phosphate pH 7.0 and 5% 2-propanol, whereby buffer B consists of 0,05 M Phosphate pH 7.0 and 20% 2-propanol. HPLC column is operated in 1 ml/min flow rate and gradient from buffer A to buffer B is achieved in 20 minutes. ADC sample is diluted in buffer A at 5 mg/ml concentration and 10 µl is injected on the HPLC column prior gradient elution.
Figure 26 shows analytical results of sample fraction characterization displayed in quantitative area of analyzed elution fractions (1A3 to 3C10), including loaded ADC (load) at 10 mg ADC/ ml CV loading and 250 mM NaCl.
Figure 27 shows deconvoluted mass spectrum for the Kadcyla^{™} sample, showing a distribution of DAR species raging from the DAR0 to DAR7 species.
Figure 28 shows deconvoluted mass spectrum for the elution fraction 3A4 sample, showing a distribution of lower DAR species.
Figure 29 shows deconvoluted mass spectrum for the elution fraction 3B12 sample, showing a distribution of higher DAR species.
Figure 30 represents elution peak of the bound ADC on the separation matrix at 60 mg ADC/ ml CV loading and 250 mM NaCl. UV adsorption signal trace in solid line and pH signal trace in dashed line.
Figure 31 shows analytical results of sample fraction characterization displayed in quantitative area of analyzed elution fractions (2A1 to 4C10), including loaded ADC (load) at 60 mg ADC/ ml CV loading and 250 mM NaCl.
Figure 32 shows elution peak of the bound ADC on the Capto^{™} MMC ImpRes chromatography resin at 30 mg ADC/ ml CV loading and 250 mM NaCl. UV adsorption signal trace in solid line and pH signal trace in dashed line.
Figure 33 shows analytical results of sample fraction characterization displayed in quantitative area of analyzed elution fractions (2B1 to 5D1), including loaded ADC (load) at 30 mg ADC/ ml CV loading and 250 mM NaCl.
Figure 34 shows elution peak of the bound ADC on the separation matrix at 10 mg ADC/ ml CV loading and 400 mM NaCl using step approach. UV adsorption signal trace in solid line and pH signal trace in dashed line.

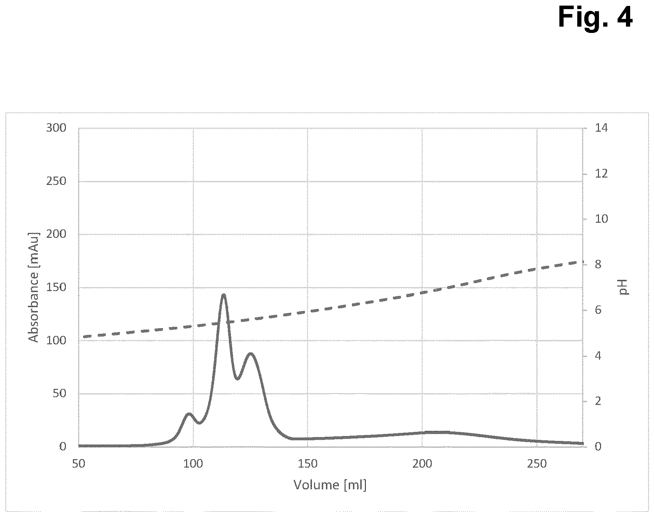

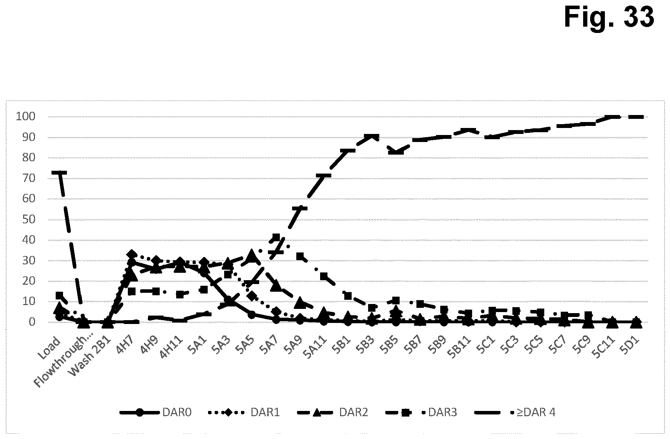

Figures 4 to 34 are further explained in the Examples.

Before describing the present invention in detail, it is to be understood that this invention is not limited to specific compositions or process steps, as such may vary. It must be noted that, as used in this specification and the appended claims, the singular form "a", "an" and "the" include plural referents unless the context clearly dictates otherwise. Thus, for example, reference to "a ligand" includes a plurality of ligands and reference to "an antibody" includes a plurality of antibodies and the like.

Unless defined otherwise, all technical and scientific terms used herein have the same meaning as commonly understood by one of ordinary skill in the art to which this invention is related. The following terms are defined for purposes of the invention as described herein.

As used herein the term "target molecule" refers to any molecule, substance or compound that shall be isolated, separated or purified from one or more other components, e.g. impurities, in a sample. Examples of target molecules are ADCs. In the production and/or purification process the target molecule is typically present in a liquid. The liquid might be water, a buffer, a non-aqueous solvent like ethanol or any mixture thereof. Beside the target molecule said liquid may comprise one or more impurities. The liquid may also be called sample. The composition of the liquid may change during production and/or purification depending on the process steps that are performed. After a chromatographic step the liquid typically comprises other solvents than before because of the eluent used in the chromatographic step. Typically only after the very last purification step the target molecule might be dried for preparing the final dosage form.

The term "antibody" refers to a protein which has the ability to specifically bind to an antigen. "Antibody" or "IgG" further refers to a polypeptide substantially encoded by an immunoglobulin gene or immunoglobulin genes, or fragments thereof, which specifically bind and recognize an analyte (antigen). The recognized immunoglobulin genes include the kappa, lambda, alpha, gamma, delta, epsilon and mu constant region genes, as well as the myriad immunoglobulin variable region genes. Light chains are classified as either kappa or lambda. Heavy chains are classified as gamma, mu, alpha, delta, or epsilon, which in turn define the immunoglobulin classes, IgG, IgM, IgA, IgD, and IgE, respectively.

An exemplary immunoglobulin (antibody) structural unit is composed of two pairs of polypeptide chains, each pair having one "light" (about 25 kD) and one "heavy" chain (about 50-70 kD), said chains being stabilized, for example, by interchain disulfide bonds. The N-terminus of each chain defines a variable region of about 100 to 110 or more amino acids primarily responsible for antigen recognition. The terms variable light chain (V L) and variable heavy chain (V H) refer to these light and heavy chains respectively.

Antibodies may be monoclonal or polyclonal and may exist in monomeric or polymeric form, for example, IgM antibodies which exist in pentameric form and/or IgA antibodies which exist in monomeric, dimeric or multimeric form. Antibodies may also include multispecific antibodies (e.g., bispecific antibodies), and antibody fragments so long as they retain, or are modified to comprise, a ligand-specific binding domain. The term "fragment" refers to a part or portion of an antibody or antibody chain comprising fewer amino acid residues than an intact or complete antibody or antibody chain. Fragments can be obtained via chemical or enzymatic treatment of an intact or complete antibody or antibody chain. Fragments can also be obtained by recombinant means. When produced recombinantly, fragments may be expressed alone or as part of a larger protein called a fusion protein. Exemplary fragments include Fab, Fab', F(ab')2, Fc and/or Fv fragments. Exemplary fusion proteins include Fc fusion proteins. According to the present invention fusion proteins are also encompassed by the term "antibody".

In some embodiments, an antibody is an Fc region containing protein, e.g., an immunoglobulin. In some embodiments, an Fc region containing protein is a recombinant protein which includes the Fc region of an immunoglobulin fused to another polypeptide or a fragment thereof. Exemplary polypeptides include, e.g., renin; a growth hormone, including human growth hormone and bovine growth hormone; growth hormone releasing factor; parathyroid hormone; thyroid stimulating hormone; lipoproteins; α- 1 -antitrypsin; insulin α-chain; insulin β-chain; proinsulin; follicle stimulating hormone; calcitonin; luteinizing hormone; glucagon; clotting factors such as factor VIIIC, factor IX, tissue factor, and von Willebrand factor; anti-clotting factors such as Protein C; atrial natriuretic factor; lung surfactant; a plasminogen activator, such as urokinase or tissue-type plasminogen activator (t-PA); bombesin; thrombin; hemopoietic growth factor; tumor necrosis factor -α and -β; enkephalinase; RANTES (regulated on activation normally T- cell expressed and secreted); human macrophage inflammatory protein (MIP-1-α); a serum albumin such as human serum albumin; Muellerian-inhibiting substance; relaxin α-chain; relaxin β-chain; prorelaxin; mouse gonadotropin-associated peptide; a microbial protein, such as β-lactamase; DNase; IgE; a cytotoxic T-lymphocyte associated antigen (CTLA) (e.g., CTLA-4); inhibin; activin; vascular endothelial growth factor (VEGF); receptors for hormones or growth factors; Protein A or D; rheumatoid factors; a neurotrophic factor such as bone- derived neurotrophic factor (BDNF), neurotrophin-3, -4, -5, or -6 (NT-3, NT-4, NT-5, or NT- 6), or a nerve growth factor such as NGF-β.; platelet-derived growth factor (PDGF); fibroblast growth factor such as αFGF and βFGF; epidermal growth factor (EGF); transforming growth factor (TGF) such as TGF-alpha and TGF-β, including TGF-βl, TGF-β2, TGF-β3, TGF-β4, or TGF-β5; insulin-like growth factor-I and -II (IGF-I and IGF-II); des(I-3)-IGF-I (brain IGF-I), insulin-like growth factor binding proteins (IGFBPs); CD proteins such as CD3, CD4, CD8, CD 19 CD20, CD34, and CD40; erythropoietin; osteoinductive factors; immunotoxins; a bone morphogenetic protein (BMP); an interferon such as interferon-α, -β, and -γ; colony stimulating factors (CSFs), e.g., M-CSF, GM-CSF, and G-CSF; interleukins (ILs), e.g., IL-I to IL-IO; superoxide dismutase; T-cell receptors; surface membrane proteins; decay accelerating factor; viral antigen such as, for example, a portion of the AIDS envelope; transport proteins; homing receptors; addressins; regulatory proteins; integrins such as CDI Ia, CDI Ib, CDI Ic, CD 18, an ICAM, VLA-4 and VCAM; a tumor associated antigen such as HER2, HER3 or HER4 receptor; and fragments and/or variants of any of the above-listed polypeptides. In addition, an antibody according to the present invention is any protein or polypeptide, fragment or variant thereof, that binds specifically to any of the above-listed polypeptides.

As used herein, and unless stated otherwise, the term "sample" refers to any composition or mixture that contains a target molecule. Samples may be derived from biological or other sources. Biological sources include eukaryotic sources like animals or humans. Preferred samples are blood or plasma samples derived from mammalians. The sample may also include diluents, buffers, detergents, and contaminating species and the like that are found mixed with the target molecule. The sample may be "partially purified" (i.e., having been subjected to one or more purification steps, such as filtration or centrifugation steps) or may be obtained directly from an organism producing the target molecule. A plasma sample is any sample comprising plasma or parts of plasma.

The term "impurity" or "contaminant" as used herein, refers to any foreign or objectionable molecule, including a biological macromolecule such as DNA, RNA, one or more host cell proteins, nucleic acids, endotoxins, lipids, impurities of synthetic origin and one or more additives which may be present in a sample containing the target molecule that is being separated from one or more of the foreign or objectionable molecules. The term "impurity" or "contaminant" as used herein can also be applied to certain immunoglobulins which need to be separated from the target molecule like immunoglobulin A which causes allergic reactions in patents as well as immunoglobulin M. Additionally, such impurity may include any reagent which is used in a step of the production and/or purification process.

The terms "purifying," "separating," or "isolating," as used interchangeably herein, refer to increasing the degree of purity of a target molecule by separating it from a composition or sample comprising the target molecule and one or more other components, e.g. impurities. Typically, the degree of purity of the target molecule is increased by removing (completely or partially) at least one impurity from the composition.

The term "chromatography" refers to any kind of technique which separates an analyte of interest (e.g. a target molecule) from other molecules present in a mixture. Usually, the target molecule is separated from other molecules as a result of differences in rates at which the individual molecules of the mixture migrate through a stationary medium or matrix under the influence of a moving phase, or in bind and elute processes. Examples for chromatographic separation processes are reversed phase chromatography, ion exchange chromatography, size exclusion chromatography, affinity chromatography, hydrophobic interaction chromatography and mixed mode chromatography.

A "buffer" is a solution that resists changes in pH by the action of its acidbase conjugate components. Various buffers which can be employed depending, for example, on the desired pH of the buffer are described in Buffers. A Guide for the Preparation and Use of Buffers in Biological Systems, Gueffroy, D., ed. Calbiochem Corporation (1975). Non- limiting examples of buffers include MES, MOPS, MOPSO, Tris, HEPES, phosphate, acetate, citrate, succinate, glycine and ammonium buffers, as well as combinations of these.

The term "matrix", "chromatography matrix", "separation matrix" as well as separation material are used interchangeably herein and refer to any kind of sorbent, support, resin or solid phase which in a separation process is used as stationary phase and separates a target molecule (e.g., an Fc region containing protein such as an immunoglobulin) from other molecules present in a mixture. Usually, the target molecule is separated from other molecules as a result of differences in rates at which the individual molecules of the mixture migrate through the matrix and interact with the matrix under the influence of a moving phase, or in bind and elute processes. The matrix typically consisting of resin particles, monolithic sorbents or a membrane can be put in columns or cartridges. Typically the matrix carries one or more types of ligands that means the matrix comprises a base matrix or base material to which one or more types of ligands are attached.

A "ligand" is or comprises a functional group and is attached to the chromatography base matrix and determines or influences the binding properties of the matrix. Preferably, the ligands are polymer chains carrying one or more, preferably a plurality of functional groups. Most preferred are ligands made of polymer chains which carry at least one functional group per monomer unit from which they are built. Examples of "ligands" include, but are not limited to, ion exchange groups, hydrophobic interaction groups, hydrophilic interaction groups, thiophilic interactions groups, metal affinity groups, affinity groups, bioaffinity groups, and mixed mode groups (combinations of the aforementioned). Preferred ligands that can be used herein include, but are not limited to, are weak ion exchange groups, such as carboxylic acid.

The term "ion-exchange" and "ion-exchange chromatography" refers to the chromatographic process in which a target molecule (e.g., an Fc region containing target protein) in a mixture interacts with a charged compound linked (such as by covalent attachment) to an ion exchange matrix such that the target molecule interacts non-specifically with the charged compound more or less than solute impurities or contaminants in the mixture. The impurities in the mixture elute from a column of the ion exchange material faster or slower than the target molecule or are bound to or excluded from the resin relative to the target molecule. "Ion-exchange chromatography" specifically includes cation exchange, anion exchange, and mixed mode ion exchange chromatography. Ion exchange chromatography can bind the target molecule (e.g., an Fc region containing target protein) followed by elution or can predominately bind the impurities while the target molecule "flows through" the column. Preferably, the ion exchange chromatography step is performed in a bind and elute mode.

The phrase "ion exchange matrix" refers to a chromatography matrix that is negatively charged (i.e. a cation exchange resin) or positively charged (i.e. an anion exchange resin). The charge may be provided by attaching one or more charged ligands to the matrix, e.g. by covalent linking. Alternatively, or in addition, the charge may be an inherent property of the matrix.

The term "cation exchange matrix" is used herein to refer to a chromatography matrix which is negatively charged, e.g. having one or more negatively charged ligands, such as carboxylic acid groups, attached thereto.

When "loading" a separation column in bind and elute mode, a buffer is used to load the sample or composition comprising the target molecule (e.g., an Fc region containing target protein) and one or more impurities onto a chromatography column (e.g an ion exchange column). The buffer has a conductivity and/or pH such that the target molecule is bound to the chromatography matrix while ideally all the impurities are not bound and flow through the column.

When "loading" a separation column to "flow through" a target molecule, a buffer is used to load the sample or composition comprising the target molecule (e.g., an Fc region containing target protein) and one or more impurities onto a chromatography column (e.g. an ion exchange column). The buffer has a conductivity and/or pH such that the target molecule is not bound to the chromatography matrix and flows through the column while ideally all the impurities are bound the column.

When "loading" a separation column to "bind and elute" a target molecule, a buffer is used to load the sample or composition comprising the target molecule (e.g., an Fc region containing target protein) and one or more impurities onto a chromatography column (e.g. an ion exchange column). The buffer has a conductivity and/or pH such that the target molecule is bound to the chromatography matrix. The separation from the one or more impurities follows with a change of conductivity and/or pH such that the target molecule is washed or eluted before or after one or more impurities. Typically the buffer in which the sample is loaded on the matrix is called loading buffer or sample buffer.

The term "equilibrating " refers to the use of a buffer to equilibrate the chromatography matrix prior to loading the target molecule. Typically, the loading buffer is used for equilibrating.

By "wash" or "washing" a chromatography matrix is meant passing an appropriate liquid, e.g. a buffer through or over the matrix. Typically washing is used to remove weakly bound contaminants from the matrix prior to eluting the target molecule and/or to remove non-bound or weakly bound target molecule after loading.

In this case, typically, the wash buffer and the loading buffer are the same. In case virus inactivation buffer is used, it is used to inactivate certain present virus prior to eluting the target molecule. In this case, typically, the virus inactivation buffer differs from loading buffer since it may contain detergent/detergents or have different properties (pH/conductivity/salts and their amounts).

Washing can also be used to remove contaminants from the matrix after the elution of the target molecule. This is done by passing an appropriate liquid, e.g. a buffer through or over the matrix after the elution of the target molecule. In this case, typically, the washing buffer differs from loading buffer. It may contain detergent/detergents or have different properties (pH/conductivity/salts and their amounts). The washing buffer is for example an acidic buffer.

To "elute" a molecule (e.g., a polypeptide of interest like Immunoglobulin G or an impurity) from a chromatography matrix is meant to remove the molecule therefrom. Elution may take place directly in flow though mode when the target molecule is eluted with the solvent front of the loading buffer or by altering the solution conditions such that a buffer different from the loading buffer competes with the molecule of interest for the ligand sites on the chromatography matrix. A non-limiting example is to elute a molecule from an ion exchange matrix by altering the ionic strength of the buffer surrounding the ion exchange material such that the buffer competes with the molecule for the charged sites on the ion exchange material.

The term "average particle size diameter" or d50 means the average particle size distribution value at 50% of the cumulative particle size distribution. Particle size is determined by laser-diffraction, preferably with Malvern 'Master Sizer.

The term "average pore size" means the average pore size distribution value at 50% of the cumulative pore size distribution.

The terms "flow-through process," "flow-through mode," and "flow-through operation," as used interchangeably herein, refer to a separation technique in which at least one target molecule (e.g., an Fc-region containing protein or an antibody) contained in a sample along with one or more impurities is intended to flow through a chromatography matrix, which usually binds the one or more impurities, where the target molecule usually does not bind (i.e., flows through) and is eluted from the matrix with the loading buffer.

The terms "bind and elute mode" and "bind and elute process," as used herein, refer to a separation technique in which at least one target molecule contained in a sample (e.g., an Fc region containing protein) binds to a suitable chromatography matrix (e.g., an ion exchange chromatography media) and is subsequently eluted with a buffer different from the loading buffer.

Antibody-drug conjugate" or "ADC" refers to antibodies, or antigen-binding fragments thereof and are conjugated to a drug such as a cytotoxic, cytostatic, and/or therapeutic agent, as described further herein below.

The term "drug" is used herein interchangeably with "payload" and refers to a chemical compound or protein or protein fragment or other cytotoxic, growth inhibitory or imaging agent which is conjugated, or to be conjugated, to the antibody. Thus in some embodiments, the drug is a small molecule. In some embodiments, the small molecule is a cytotoxic agent or an imaging agent. In some embodiments, the cytotoxic agent is selected from the group consisting of an anthracycline, an auristatin, a dolastatin, a duocarmycin, an enediyne, a geldanamycin, a maytansine, a puromycin, a taxane, a vinca alkaloid, SN-38, a tubulysin, a hemiasterlin and stereoisomers, isosteres, analogs, or derivatives thereof. In some embodiments, the drug is a biocompatible polymer or a polypeptide. "Drug" as used herein is not to be construed as limited to classical chemical therapeutic agents. For example, the drug moiety may be a protein or polypeptide possessing a desired biological activity. Preferably the drug is a small molecule.

The term "drug to antibody ratio" (abbreviated as "DAR") refers to the number of drug molecules conjugated per antibody. Compositions, batches, and/or formulations of a plurality of antibody-drug conjugates may be characterized by an average DAR. DAR and average DAR can be determined by various conventional means such as UV spectroscopy, mass spectroscopy, ELISA assay, radiometric methods, hydrophobic interaction chromatography (HIC), electrophoresis and HPLC.

The term "cytotoxic agent" as used herein refers to a substance that inhibits or prevents the function of cells and/or causes destruction of cells. The term is intended to include radioactive isotopes (e.g., At211, 1131, 1125, Y90, Rel86, Rel88, Sml53, Bi212, P32 and radioactive isotopes of Lu), chemotherapeutic agents, enzymes and fragments thereof such as nucleolytic enzymes, antibiotics, and toxins such as small molecule toxins or enzymatically active toxins of bacterial, fungal, plant or animal origin, including fragments and/or variants thereof, and the various antitumor or anticancer agents disclosed below.

Other cytotoxic agents are described below. A tumoricidal agent causes destruction of tumor cells. A "chemotherapeutic agent" is a chemical compound useful in the treatment of cancer. Examples of chemotherapeutic agents include alkylating agents such as thiotepa and CYTOXAN(R) cyclosphosphamide; alkyl sulfonates such as busulfan, improsulfan and piposulfan; aziridines such as benzodopa, carboquone, meturedopa, and uredopa; ethylenimines and methylamelamines including altretamine, triethylenemelamine, trietylenephosphoramide, triethiylenethiophosphoramide and trimethylolomelamine; acetogenins (especially bullatacin and bullatacinone); delta-9-tetrahydrocannabinol (dronabinol, MARINOL(R)); beta-lapachone; lapachol; colchicines; betulinic acid; a camptothecin (including the synthetic analogue topotecan (HYCAMTIN(R)), CPT-11 (irinotecan, CAMPTOSAR(R)), acetylcamptothecin, scopolectin, and 9-aminocamptothecin); bryostatin; callystatin; CC-1065 (including its adozelesin, carzelesin and bizelesin synthetic analogues); podophyllotoxin; podophyllinic acid; teniposide; cryptophycins (particularly cryptophycin 1 and cryptophycin 8); dolastatin; duocarmycin (including the synthetic analogues, KW-2189 and CB1-TM1); eleutherobin; pancratistatin; a sarcodictyin; spongistatin; nitrogen mustards such as chlorambucil, chlornaphazine, cholophosphamide, estramustine, ifosfamide, mechlorethamine, mechlorethamine oxide hydrochloride, melphalan, novembichin, phenesterine, prednimustine, trofosfamide, uracil mustard; nitrosureas such as carmustine, chlorozotocin, fotemustine, lomustine, nimustine, and ranimnustine; antibiotics such as the enediyne antibiotics (e. g., calicheamicin, especially calicheamicin gammall and calicheamicin omegall (see, e.g., Agnew, Chem Intl. Ed. Engl., 33: 183-186 (1994)); dynemicin, including dynemicin A; an esperamicin; as well as neocarzinostatin chromophore and related chromoprotein enediyne antiobiotic chromophores), aclacinomysins, actinomycin, authramycin, azaserine, bleomycins, cactinomycin, carabicin, carminomycin, carzinophilin, chromomycinis, dactinomycin, daunorubicin, detorubicin, 6-diazo-5-oxo-L-norleucine, ADRIAMYCIN(R) doxorubicin including morpholino-doxorubicin, cyanomorpholino-doxorubicin, 2-pyrrolino-doxorubicin and deoxydoxorubicin), epirubicin, esorubicin, idarubicin, marcellomycin, mitomycins such as mitomycin C, mycophenolic acid, nogalamycin, olivomycins, peplomycin, potfiromycin, puromycin, quelamycin, rodorubicin, streptonigrin, streptozocin, tubercidin, ubenimex, zinostatin, zorubicin; anti-metabolites such as methotrexate and 5-fluorouracil (5-FU); folic acid analogues such as denopterin, methotrexate, pteropterin, trimetrexate; purine analogs such as fludarabine, 6-mercaptopurine, thiamiprine, thioguanine; pyrimidine analogs such as ancitabine, azacitidine, 6-azauridine, carmofur, cytarabine, dideoxyuridine, doxifluridine, enocitabine, floxuridine; androgens such as calusterone, dromostanolone propionate, epitiostanol, mepitiostane, testolactone; antiadrenals such as aminoglutethimide, mitotane, trilostane; folic acid replenisher such as frolinic acid; aceglatone; aldophosphamide glycoside; aminolevulinic acid; eniluracil; amsacrine; bestrabucil; bisantrene; edatraxate; defofamine; demecolcine; diaziquone; elfornithine; elliptinium acetate; an epothilone; etoglucid; gallium nitrate; hydroxyurea; lentinan; lonidainine; maytansinoids such as maytansine and ansamitocins; mitoguazone; mitoxantrone; mopidanmol; nitraerine; pentostatin; phenamet; pirarubicin; losoxantrone; 2-ethylhydrazide; procarbazine; PSK(R) polysaccharide complex (JHS Natural Products, Eugene, OR); razoxane; rhizoxin; sizofiran; spirogermanium; tenuazonic acid; triaziquone; 2,2',2"-trichlorotriethylamine; trichothecenes (especially T-2 toxin, verracurin A, roridin A and anguidine); urethan; vindesine (ELDISINE(R), FILDESIN(R)); dacarbazine; mannomustine; mitobronitol; mitolactol; pipobroman; gacytosine; arabinoside ("Ara-C"); thiotepa; taxoids, e.g., TAXOL(R) paclitaxel (Bristol-Myers Squibb Oncology, Princeton, N.J.), ABRAXANETM Cremophor-free, albumin-engineered nanoparticle formulation of paclitaxel (American Pharmaceutical Partners, Schaumberg, Illinois), and TAXOTERE(R) doxetaxel (Rhone-Poulenc Rorer, Antony, France); chloranbucil; gemcitabine (GEMZAR(R)); 6-thioguanine; mercaptopurine; methotrexate; platinum analogs such as cisplatin and carboplatin; vinblastine (VELBAN(R)); platinum; etoposide (VP- 16); ifosfamide; mitoxantrone; vincristine (ONCOVIN(R)); oxaliplatin; leucovovin; vinorelbine (NAVELBINE(R)); novantrone; edatrexate; daunomycin; aminopterin; ibandronate; topoisomerase inhibitor RFS 2000; difluorometlhylornithine (DMFO); retinoids such as retinoic acid; capecitabine (XELODA(R)); pharmaceutically acceptable salts, acids or derivatives of any of the above; as well as combinations of two or more of the above such as CHOP, an abbreviation for a combined therapy of cyclophosphamide, doxorubicin, vincristine, and prednisolone, and FOLFOX, an abbreviation for a treatment regimen with oxaliplatin (ELOXATINTM) combined with 5-FU and leucovovin.

Also included in this definition are anti-hormonal agents that act to regulate, reduce, block, or inhibit the effects of hormones that can promote the growth of cancer, and are often in the form of systemic, or whole-body treatment. They may be hormones themselves. Examples include antiestrogens and selective estrogen receptor modulators (SERMs), including, for example, tamoxifen (including NOLVADEX(R) tamoxifen), EVISTA(R) raloxifene, droloxifene, 4-hydroxytamoxifen, trioxifene, keoxifene, LY1 17018, onapristone, and FARESTON(R) toremifene; anti-progesterones; estrogen receptor down-regulators (ERDs); agents that function to suppress or shut down the ovaries, for example, leutinizing hormone-releasing hormone (LHRH) agonists such as LUPRON(R) and ELIGARD(R) leuprolide acetate, goserelin acetate, buserelin acetate and tripterelin; other anti-androgens such as flutamide, nilutamide and bicalutamide; and aromatase inhibitors that inhibit the enzyme aromatase, which regulates estrogen production in the adrenal glands, such as, for example, 4(5)-imidazoles, aminoglutethimide, MEGASE(R) megestrol acetate, AROMASIN(R) exemestane, formestanie, fadrozole, RIVISOR(R) vorozole, FEMARA(R) letrozole, and ARIMIDEX(R) anastrozole. In addition, such definition of chemotherapeutic agents includes bisphosphonates such as clodronate (for example, BONEFOS(R) or OSTAC(R)), DIDROCAL(R) etidronate, NE-58095, ZOMETA(R) zoledronic acid/zoledronate, FOSAMAX(R) alendronate, AREDIA(R) pamidronate, SKELID(R) tiludronate, or ACTONEL(R) risedronate; as well as troxacitabine (a 1,3-dioxolane nucleoside cytosine analog); antisense oligonucleotides, particularly those that inhibit expression of genes in signaling pathways implicated in abherant cell proliferation, such as, for example, PKC-alpha, Raf, H-Ras, and epidermal growth factor receptor (EGF-R); vaccines such as THERATOPE(R) vaccine and gene therapy vaccines, for example, ALLOVECTIN(R) vaccine, LEUVECTIN(R) vaccine, and VAXID(R) vaccine; LURTOTECAN(R) topoisomerase 1 inhibitor; ABARELIX(R) rmRH; lapatinib ditosylate (an ErbB-2 and EGFR dual tyrosine kinase small-molecule inhibitor also known as GW572016); and pharmaceutically acceptable salts, acids or derivatives of any of the above.

A "growth inhibitory agent" when used herein refers to a compound or composition which inhibits growth of a cell, especially a TAT-expressing cancer cell, either in vitro or in vivo. Thus, the growth inhibitory agent may be one which significantly reduces the percentage of TAT-expressing cells in S phase. Examples of growth inhibitory agents include agents that block cell cycle progression (at a place other than S phase), such as agents that induce Gl arrest and M-phase arrest. Classical M-phase blockers include the vincas (vincristine and vinblastine), taxanes, and topoisomerase II inhibitors such as doxorubicin, epirubicin, daunorubicin, etoposide, and bleomycin. Those agents that arrest Gl also spill over into S-phase arrest, for example, DNA alkylating agents such as tamoxifen, prednisone, dacarbazine, mechlorethamine, cisplatin, methotrexate, 5- fluorouracil, and ara-C. Further information can be found in The Molecular Basis of Cancer, Mendelsohn and Israel, eds., Chapter 1, entitled "Cell cycle regulation, oncogenes, and antineoplastic drugs" by Murakami et al. (WB Saunders: Philadelphia, 1995), especially p. 13. The taxanes (paclitaxel and docetaxel) are anticancer drugs both derived from the yew tree. Docetaxel (TAXOTERE(R), Rhone-Poulenc Rorer), derived from the European yew, is a semisynthetic analogue of paclitaxel (TAXOL(R), Bristol-Myers Squibb). Paclitaxel and docetaxel promote the assembly of microtubules from tubulin dimers and stabilize microtubules by preventing depolymerization, which results in the inhibition of mitosis in cells.

The present invention is directed to the purification of ADCs. As defined above an ADC is an antibody-conjugated drug formed by covalently binding an antibody to a toxin, typically a small molecule toxin, preferably via a linker, involving lysine coupling, light and heavy chain reductive disulfide coupling, or other coupling methods. The antibody may be any kind of antibody or antibody fragment, e.g. a polyclonal antibody or a monoclonal antibody, preferably a monoclonal antibody. Examples of small molecule toxins are the maytansin derivative DM1 (N2'-deacetyl-N2'-3-mercapto-1 oxopropyl)-maytansin), DM4 (N2'-deacetyl-N2'-4) -Methyl-4-mercapto-1oxopentyl)-maytansine and its analogues, the dolphin toxin derivative MMAE (Monomethyl Auristatin E, methyl auristatin E), MMAF (Monomethyl Auristatin F, methyl Auristatin F) and its analogs. An exemplary linker is SMCC (4-(N-maleimidomethyl)cyclohexane-1-carboxylic acid succinimide ester).

The present invention provides separation materials, also called resins, comprising a base matrix to which polymer chains are covalently attached. The polymer chains are made of monomers which comprise an amino acid and a polymerizable double bond.

The present invention further provides methods for the separation and purification of ADCs, e.g. for the separation of ADCs with different DARs or for removing high molecular weight species, specifically aggregates, from antibody drug conjugate (ADC) preparations. The present invention is useful for large scale purification of antibody drug conjugate preparations. In particular aspects of the invention, it is beneficial to eliminate ADCs having a high DAR (DAR > 6) which are more hydrophobic and demonstrate faster clearance, which may contribute to higher toxicity and lower the therapeutic index (Tl). In other aspects of the invention, it is beneficial to eliminate ADCs having a low DAR (DAR < 2) which contribute little to efficacy, however, provide an increase in the amounts of unconjugated antibody which may compete with the ADC for the target antigen, and lead to a lower Tl. In another aspect of the invention, it is beneficial to eliminate ADCs having a high DAR (DAR > 6) and ADCs having a low DAR (DAR < 2). In one embodiment of the invention the purified ADC contains drug loading (drug to antibody ratio, DAR) between 3.5 and 4.5 and in another embodiment between 3 and 5.

The separation materials of the present invention comprise a base material to which tentacle-like structures are grafted.

The base material, also called base matrix, contains reactive groups which are accessible to the graft-polymerisation reaction, in particular OH groups, preferably aliphatic OH groups. Base materials can therefore also be prepared, for example, from organic polymers. Organic polymers of this type can be polysaccharides, such as agarose, dextrans, starch, cellulose, etc., or synthetic polymers, such as poly(acrylamides), poly(methacrylamides), poly(acrylates), poly(methacrylates), hydrophilically substituted poly(alkyl allyl ethers), hydrophilically substituted poly(alkyl vinyl ethers), poly(vinyl alcohols), poly(styrenes) and copolymers of the corresponding monomers. These organic polymers can preferably also be employed in the form of a crosslinked hydrophilic network. This also includes polymers made from styrene and divinylbenzene, which can preferably be employed, like other hydrophobic polymers, in a hydrophilized form.

Alternatively, inorganic materials, such as silica, zirconium oxide, titanium dioxide, aluminium oxide, etc., can be employed as base materials. It is equally possible to employ composite materials, i.e., for example, particles which can themselves be magnetised by copolymerisation of magnetisable particles or of a magnetisable core.

However, preference is given to the use of hydrophilic base materials which are stable to hydrolysis or can only be hydrolysed with difficulty since the materials according to the invention should preferably withstand alkaline cleaning or regeneration at e.g. basic pH over an extended use duration.

The base materials may consist of irregularly shaped or spherical particles, whose particle size can be between 2 and 1000 µm. Preference is given to particle sizes between 3 and 300 µm.

The base materials may, in particular, be in the form of non-porous or preferably porous particles. The pore sizes can be between 2 and 300 nm. Preference is given to pore sizes between 5 and 200 nm.

The base materials may equally also be in the form of membranes, fibres, hollow fibres, coatings or monolithic mouldings. Monolithic mouldings are, preferably porous, three-dimensional bodies, for example in cylindrical form.

In a preferred embodiment, the base material is a copolymer formed by copolymerisation of at least one compound from the group a) and b) with
a) at least one hydrophilically substituted alkyl vinyl ether of the formula I 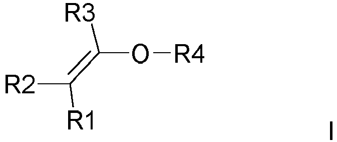
   where R1, R2, R3, independently of one another, can be H or C1 to C6 alkyl, preferably H or -CH₃,
   and R4 is a radical which carries at least one hydroxyl group and
b) at least one crosslinking agent conforming to formula II and/or III and/or IV with 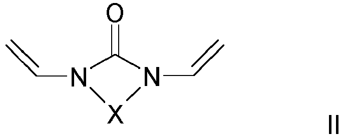 where X is a divalent alkyl radical having 2 to 5 C atoms, preferably 2 or 3 C atoms, in which one or more methylene groups which are not adjacent and are not located in the direct vicinity of N may be replaced by O, C=O, S, S=O, SO₂, NH, NOH or N and one or more H atoms of the methylene groups may be substituted, independently of one another, by hydroxyl groups, C1-C6-alkyl, halogen, NH₂, C5-C10-aryl, NH-(C1-C8)-alkyl, N-(C1-C8)-alkyl_{2,} C1-C6-alkoxy or C1-C6-alkyl-OH, and 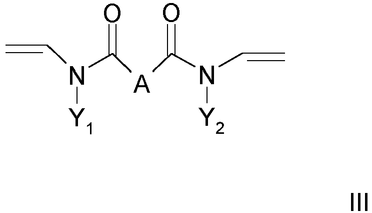 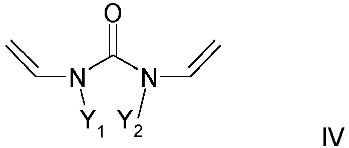
   where Y1 and Y2 in formula III and IV are, independently of one another,
   C1 to C10 alkyl or cycloalkyl, where one or more non-adjacent methylene groups or methylene groups which are not located in the direct vicinity of N may be replaced by O, C=O, S, S=O, SO₂, NH, NOH or N and one or more H of the methylene groups may be substituted, independently of one another, by hydroxyl groups, C1-C6-alkyl, halogen, NH₂, C5-C10-aryl, NH(C1-C8)alkyl, N(C1-C8)alkyl_{2,} C1-C6-alkoxy or C1-C6-alkyl-OH,
   or C6 to C18 aryl, where one or more H in the aryl system may be substituted, independently of one another, by hydroxyl groups, C1-C6-alkyl, halogen, NH₂, NH(C1-C8)alkyl, N(C1-C8)alkyl_{2,} C1-C6-alkoxy or C1-C6-alkyl-OH and
   A is a divalent alkyl radical having 2 to 5 C atoms, preferably 2 or 3 C atoms, in which one or more non-adjacent methylene groups or methylene groups which are not located in the direct vicinity of N may be replaced by O, C=O, S, S=O, SO₂, NH, NOH or N and one or more H of the methylene groups may be substituted, independently of one another, by hydroxyl groups, C1-C6-alkyl, halogen, NH₂, C5-C10-aryl, NH(C1-C8)alkyl, N(C1-C8)alkyl_{2,} C1-C6-alkoxy or C1-C6-alkyl-OH.

R4 in formula I is typically an alkyl radical, a cycloaliphatic radical or an aryl radical which carries at least one hydroxyl group.

In a very preferred embodiment the matrix is formed by copolymerisation of a hydrophilically substituted alkyl vinyl ether employed selected from the group of 1,4-butanediol monovinyl ether, 1,5-pentanediol monovinyl ether, diethylene glycol monovinyl ether or cyclohexanedimethanol monovinyl ether and divinylethyleneurea (1,3-divinylimidazolin-2-one) as crosslinking agent.

An example of a suitable commercially available vinylether based base material is Eshmuno^{®}, Merck KGaA, Germany.

To the surfaces of the base material linear polymer chains are covalently bonded so that a separation material is generated whereby
a) the base material contains preferably aliphatic hydroxyl groups,
b) the polymers are covalently bonded to the base material,
c) the polymers comprise amino acid residues
d) the monomer units of the polymers are linked in a linear manner

The actual separation material comprising the base material and the covalently attached linear polymer chains can be prepared in various ways. In the case of "grafting onto", polymer chains must firstly be formed from the monomers and bound to the surface in a second step. In the case of "grafting from", a polymerisation reaction is initiated on the surface, and the graft polymer is built up directly from individual monomers. Other polymerisation methods which allow binding to the surface of the support material can also be employed.

Preference is given to the "grafting from" method and particular preference is given to variants in which only a few by-products, such as a non-covalently bonded polymer, which have to be separated off are formed. Processes with controlled free-radical polymerisation, such as, for example, the method of atom-transfer free-radical polymerisation (ATRP), appear particularly suitable. Here, an initiator group is covalently bonded to the support surface in the desired density in a first step. An initiator group can be, for example, a halide bonded via an ester function, as in a 2-bromo-2-methylpropionic acid ester. The graft polymerisation is carried out in a second step in the presence of copper(I) salts.

A very preferred one-step graft polymerisation reaction suitable for the production of the separation materials of the present invention can be initiated by cerium(IV) on a hydroxyl-containing support, without the support having to be activated.

This cerium(IV) initiated grafting is preferably carried out in accordance with EP 0 337 144 or US 5,453,186. The chain produced is linked to the base material via a monomer unit. To this end, the base material according to the invention is suspended in a solution of monomers, preferably in an aqueous solution. The grafting-on of the polymeric material is effected in the course of a conventional redox polymerisation with exclusion of oxygen. The polymerisation catalyst employed is cerium(IV) ions, since this catalyst forms free-radical sites on the surface of the base material, from which the graft polymerisation of the monomers is initiated. This reaction is normally carried out in dilute mineral acids. In order to carry out this graft polymerisation, the acid is usually employed in an aqueous solution with a concentration in the range from 1 to 0.00001 mol/l, preferably from 0.1 to 0.001 mol/l. Very particular preference is given to the use of dilute nitric acid, which is employed with a concentration in the range from 0.1 to 0.001 mol/l.

For the preparation of the separation materials according to the invention, the monomers are normally added in excess to the base material. Typically, 0.05 to 100 mol of total monomer are employed per litre of sedimented polymer material, preferably 0.05-25 mol/l are employed.

The polymerisation is terminated by termination reactions involving the cerium salts. For this reason, the (average) chain length can be influenced by the concentration ratios of the base material, the initiator and the monomers. Furthermore, uniform monomers or also mixtures of different monomers can be employed; in the latter case, grafted copolymers are formed.

The monomers to be favourably used for the preparation of the separation materials according to the present invention are those according to formula V 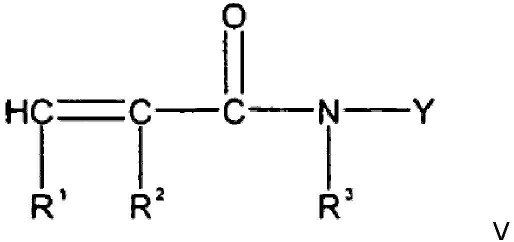
with R¹, R² and Y being independently from each other H or CH₃, preferably H.
R³ being -CHCOOMR⁴ 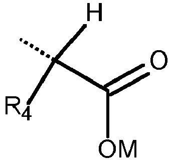
with R⁴ being C1 to C4 alkyl, like methyl, ethyl, propyl, isopropyl, butyl, isobutyl, preferably isopropyl and isobutyl, very preferred isobutyl, or C1 to C4 perfluoroalkyl
and M being H, Na, K, or NH₄⁺.

Perfluoroalkyl means that all H atoms of the alkyl residue are substituted by F atoms.

Exemplary, preferred structures of formula V are Va 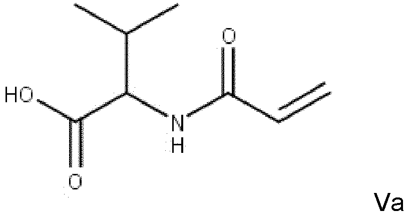
with R¹, R² and Y being H
R³ being -CHCOOMR⁴
with R⁴ being isopropyl
and M being H
as well as Formula Vb 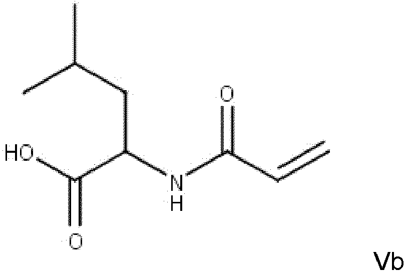
with R¹, R² and Y being H
R³ being -CHCOOMR⁴
with R⁴ being isobutyl
and M being H

Such monomers can also be described as acryloylvalin (formula Va), acryloylleucine (formula Vb), acryloylalanine, acryloylnorleucine, methacryloylvalin, methacryloylleucine, methacryloylalanine, methacryloylnorleucine, dimethacryloylvalin, dimethacryloylleucine, dimethacryloylalanine, dimethacryloylnorleucine, whereby acryloylvalin and acryloylleucine are preferred, acryloylleucine is especially preferred.

The separation materials according to the present invention preferably only contain tentacle-like linear polymer structures grafted onto the base material that are built from monomers according to formula V. Preferably, they contain linear polymers that are only build by one type of monomer according to formula V.

But it is also possible that the linear polymers are built by co-polymerization of two or more different monomers according to formula V. It is also possible that the linear polymers are built by co-polymerization of one or more different monomers according to formula V and one or more other polymerizable monomers like other acrylamides, methacrylates, acrylates, methacrylates etc. which are functionalized e.g. with ionic, hydrophilic or hydrophobic groups.

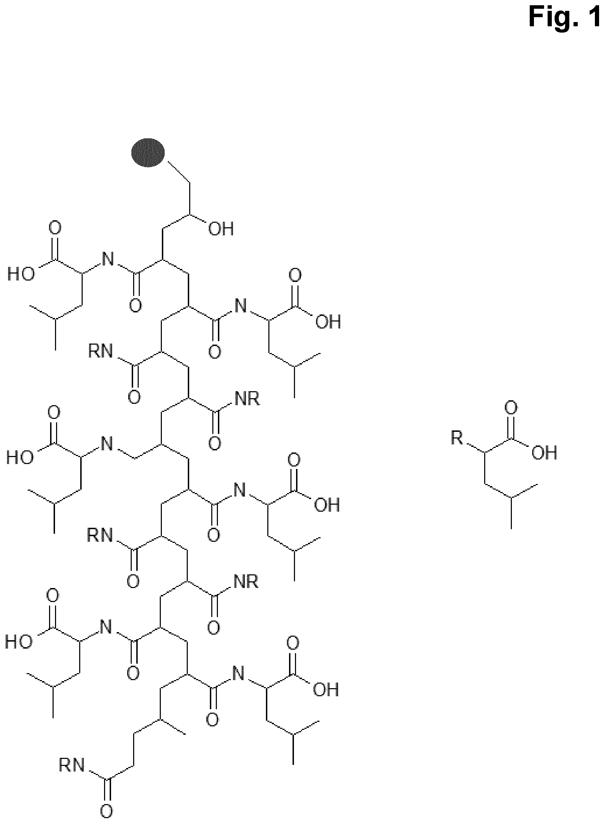

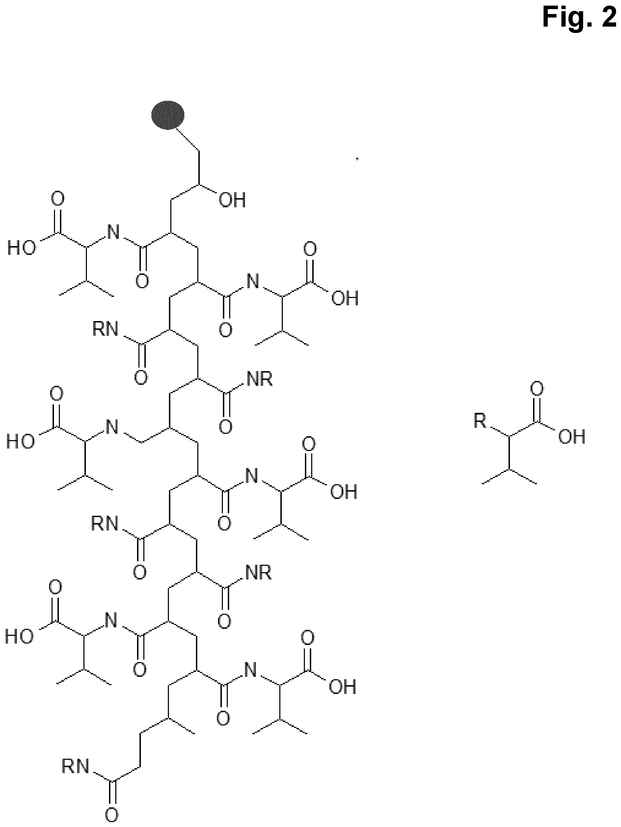

Exemplary structures of the separation materials according to the present invention are shown in Figures 1 and 2. Figure 1 shows the base material (dot) that is functionalized with a linear polymer built by polymerization of acryloylleucine monomers. Additionally, for clarification, the carboxy-alkyl endgroup of each polymer unit is shown. Figure 2 shows the base material (dot) that is functionalized with a linear polymer built by polymerization of acryloylvaline monomers. Additionally, for clarification, the carboxy-alkyl endgroup of each polymer unit is shown.

In a preferred embodiment, the separation material of the present invention is an ion exchange material with varying amount of ionic density window ranging from 10-1200µeq/g, where in more preferred embodiment the ionic density window is between 400 - 900 µeq/g.

In a preferred embodiment, the ion exchange material can contain ion exchange functional groups and hydrophobic functional groups, where in more preferred embodiment both functional groups are on a single surface functional unit resulting from the one monomeric unit that has been built into the polymeric chain, where in the most preferred embodiment the functional groups are part of an amino acid residue.

The present invention is further directed to a chromatography column comprising the above described separation materials according to the present invention. Chromatography columns are known to a person skilled in the art. They typically comprise cylindrical tubes or cartridges filled with the separation materials as well as filters and/or means for fixing the separation materials in the tube or cartridge and connections for solvent delivery to and from the tube. The size of the chromatography column varies depending on the application, e.g. analytical or preparative.

The materials according to the invention can also be described as base materials provided with separation effectors. They can be used for the selective, partially selective or non-selective binding or adsorption of one or more target components with the aim of separation out of a sample liquid, or for the selective, partially selective or non-selective binding or adsorption of one or more secondary components with the aim of separation of the secondary component out of a matrix, the isolation, enrichment and/or depletion of biopolymers from natural sources, the isolation, enrichment and/or depletion of biopolymers from recombinant sources, the isolation, enrichment and/or depletion of proteins and peptides, the isolation, enrichment and/or depletion of monoclonal and polyclonal antibodies, the isolation, enrichment and/or depletion of viruses, the isolation, enrichment and/or depletion of host cell proteins, the isolation, enrichment and/or depletion of ADCs. Preferred target molecules are ADCs.

The target molecules are separated from at least one or more other substances from a sample, where the sample which comprises the target molecule is dissolved in a liquid, which is brought into contact with the material according to the invention. Contact times are usually in the range from 30 seconds to 24 hours. It is advantageous to work in accordance with the principles of liquid chromatography by passing the liquid through a chromatography column which contains the separation material according to the invention. The liquid can run through the column merely through its gravitational force or be pumped through by means of a pump. An alternative method is batch chromatography, in which the separation material is mixed with the liquid by stirring or shaking for as long as the target molecules or biopolymers need to be able to bind to the separation material. It is likewise possible to work in accordance with the principles of the chromatographic fluidised bed by introducing the liquid to be separated into, for example, a suspension comprising the separation material, where the separation material is selected so that it is suitable for the desired separation owing to its high density and/or a magnetic core.

If the chromatographic process is run in the bind and elute mode, the target molecule binds to the separation material according to the invention. The separation material can subsequently be washed with a wash buffer, which preferably has the same ion strength and the same pH as the liquid in which the target molecule is brought into contact with the separation material. The wash buffer removes all substances which do not bind to the separation material. Further washing steps with other suitable buffers may follow without desorbing the target molecule. The desorption of the bound target molecule is carried out by changing the ion strength in the eluent and/or by changing the pH in the eluent and/or by changing the solvents. The target molecule can thus be obtained in a purified and concentrated form in the eluent. The target molecule usually has a purity of 70 percent to 99 percent, preferably 85 percent to 99 percent, particularly preferably 90 percent -99 percent, after desorption.

However, if the chromatographic process is run in the flow-through mode, the target molecule remains in the liquid, but other accompanying substances bind to the separation material. The target molecule is then obtained directly by collecting the column eluate in through-flow. It is known to the person skilled in the art how he has to adapt the conditions, in particular the pH and/or the conductivity, in order to bind a specific biopolymer to a separation material, or whether it is advantageous for the purification task not to bind the target molecule.

The present invention is preferably directed to the use of the above described separation materials according to the present invention for the separation and purification of ADCs and to a method for purification and/or separation of ADCs by liquid chromatography which comprises contacting the separation material according to the present invention with a sample comprising one or more ADCs under acidic conditions.

Unexpectedly, we have found, that the ion exchange material according to the present invention can be used for the purification of ADC's enabling an efficient DAR (drug antibody ratio) species separation at > 10 mg ADC/ml material capacities, where in more preferred embodiment the capacity is between 10 - 80 mg/ml. Moreover, this innovative ion exchange material can be used in high conductivity >10 mS/cm, where in more preferred embodiment the conductivity is between 10 - 40 mS/cm. Surprisingly, it is possible to separate each individual DAR species, regardless their conjugation procedure preferably using a pH change from 4 - 7 to a pH above 9, preferably between 9 and 11, most preferred to around pH 10 in gradient or step mode. Moreover, the application of this separation material showed significant economic advantages compared to hydrophobic interaction or hydroxy apatite materials, assuring > 90% product recovery rates. Additionally, the application is not limited to the separation of different DAR species, but can be used to remove not conjugated mAb's from the conjugated ones. Moreover, the application of the separation material is not limited to the separation of different DAR species, but can be used to remove not conjugated toxic molecules.

Additionally, the application of this material is not limited to bind and elute applications, but can be used in a flow-through mode, resulting in higher DAR species adsorption on the ion exchange material using buffers having pH < 7 and/or high conductivity ( > 20mS/cm). Further, surprisingly only this ion exchange material, that primary consists of amino acids covalently attached to the material, has the necessary selectivity for the DAR species, where in more preferred embodiment these amino acids are valine or leucine, including combinations and derivatives thereof, most preferred is leucine.

Further, the present invention provides a chromatography based ADC purification step which can be regenerated and is applicable in a wide operation window, e.g. pH 3 - 10; conductivity 1 mS/cm - 50 mS/cm.

In a preferred embodiment, the ion exchange material according to the present invention is incorporated into a chromatography column and used for ADC purification processes in a bind and elute mode for separating DAR species.

In a preferred embodiment, the ion exchange material according to the present invention is incorporated into a chromatography column and used for ADC purification processes in a bind and elute mode separating DAR species, where the operation window span is between pH 2 - pH 10, where in more preferred embodiment the pH is between 4-7.

In a preferred embodiment, the ion exchange material according to the present invention is incorporated into a chromatography column and used for ADC purification processes in a bind and elute mode separating DAR species, where solvent pH elution is used to recover the bound components.

In a preferred embodiment, the ion exchange material according to the present invention is incorporated into a chromatography column and used for ADC purification processes in a bind and elute mode for separating DAR species, where the binding window span is between 10 mg ADC/ml material to 100 mg ADC/ml material, where in more preferred embodiment the binding window span is between 20 mg ADC/ml material to 80 mg ADC/ml material.

In a preferred embodiment, the ion exchange material according to the present invention is incorporated into a chromatography column and used for ADC purification processes in a bind and elute mode for separating DAR species, where the conductivity window span is between 1 mS/cm - 50mS/cm, where in the more preferred embodiment is conductivity range between 2-30 mS/cm.

In another preferred embodiment, the ion exchange material according to the present invention is incorporated into a chromatography column and used for ADC purification processes in a flow-through mode separating DAR species, where the low DAR species are in flow-through and higher DAR species bind to material.

In a preferred embodiment, ion exchange material can be sized to various particle sizes ranging from 1 µm - 200 µm, whereby in a more preferred embodiment the average particle size is between 20 - 63 µm.

In a preferred embodiment, the ion exchange material according to the present invention can have various pore sizes ranging from 4 nm - 1500 nm, whereby in a more preferred embodiment the average pore size is between 10 - 120 nm, and in a most preferred embodiment the average pore size is between 40 - 110 nm.

The present invention is further illustrated by the following figures and examples, however, without being restricted thereto.

### Examples

The following examples represent practical applications of the invention.

### 1. Synthesis of separation material

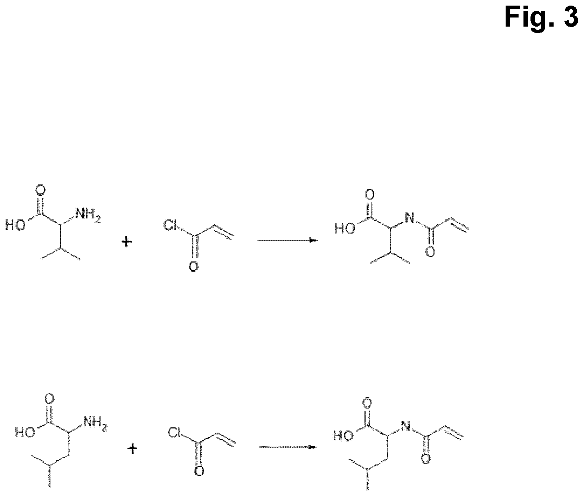

For the preparation of the monomers to be used for the grafting from process the amino acids, e.g. valine, leucine or alanine, are dissolved in VE water and the pH is adjusted to a pH above 13 by adding NaOH (32%) . At a temperature between 0 - 5 °C the acrylic compound like acrylic acid chloride or acrylic acid is added and the mixture is stirred for one hour. The reaction scheme for valine and leucine with acrylic acid chloride is shown in Figure 3.

Then the pH is adjusted to about pH 2.2 by adding nitric acid. Afterwards the OH containing base material is added, e.g. Eshmuno^{®} particles.

Polymerization is started by adding Cerium (IV) nitrate. The reaction takes place for 4 hours at 30 - 50 °C.

After polymerization reaction the non-reacted components and starter are removed by extensive washing using acidic, basic and solvent mixtures at room or elevated temperatures.

For the preparation of a separation material carrying perfluoroalkylated endgroups, hexafluoro-valine is dissolved in VE water and the pH is adjusted to a pH above 13 by adding NaOH (32%). At a temperature between 0-5 °C the acrylic compound like acrylic acid chloride or acrylic acid is added and the mixture is stirred for one hour. Then the pH is adjusted to about pH 2.2 by adding nitric acid. Afterwards the OH containing base material is added, e.g. Eshmuno^{®} particles. Polymerization is started by adding Cerium (IV) nitrate. The reaction takes place for 4 hours at 30-50 °C.

### 2.

The ion exchange material as prepared according to Example 1 (e.g. with the average particle size between 20 - 63 µm, the average pore size between 40 - 110 nm and an ionic density between 400 - 900 µeq/g) was evaluated for its ability to separate ADC species of commercially available drug Adcetris^{®}. The ion exchange material was packed in a chromatographic column of 5 × 100 mm dimensions with asymmetry between 0,8 - 1,2 and >3000 plates/m. After packing the ion exchange material, the obtained chromatographic column was cleaned with 1 M NaOH solution for 30 minutes and preequilibrated with loading buffer solution having pH of 4.5 and 300 mM NaCl (e.g. 24 mS/cm). The buffer solution contained combination of salts such as sodium dihydrogen phosphate, TRIS, glycine to obtain the pH of 4.5. The same solution was used to dissolve the lyophilized Adcetris^{®} sample till 1 mg/ml concentration. This solution was loaded on the prepared chromatographic column till 10 mg Adcetris^{®} loading was reached for 1 ml resin. These steps and the following steps were performed at 150 cm/h buffer velocity. After loading 10 mg/ml Adcetris^{®}, the chromatographic column was washed with pH 4.5 buffer solution and then eluted used gradient elution with buffer having pH of 8.5 and 300 mM NaCl. This elution buffer was prepared using different salts such as sodium dihydrogen phosphate, TRIS, glycine. The conductivity and pH values were traced during the experimental set-up, showing that the Adcetris^{®} elution from the column was achieved due to the pH change during the gradient elution. The sample elution was fractionated and obtained fractions were evaluated of line using two different methods for the DAR species identification. The first method of choice was analytical hydrophobic interaction chromatography using mAbPac HIC-Butyl column with 4.6 × 100 mm dimensions from Thermo Fisher Scientific. The chromatographic separation of fractionated samples enabled us to identify different DAR species and the quality thereof based on different elution window for each specie (e.g. the elution of DAR0 species was between 7-9 minute, the elution of DAR2 species between 10-14 minute, the elution of DAR4 species between 15-18 minute and the elution of DAR6 species between 19-22 minute).

The example chromatographic experiment of 10 mg/ml Adcetris^{®} loading is shown in Figure 4, where UV adsorption signal trace in solid line and pH signal trace in dashed line. The fractions were collected and were analyzed using analytical HIC (hydrophobic interaction chromatography) HPLC and the amount of different DARs calculated using the quantitative area (Figure 4). Figure 5 illustrates the separation of antibody drug conjugate species during a linear elution gradient on an analytical HPLC column. Five consecutive elution pools are identified in the UV signal trace, representing five different antibody drug conjugate species: peak 1 represents antibody drug conjugate species without drug, peak 2 represents antibody drug conjugate species with 2 drug molecules conjugated to a single antibody molecule, peak 3 represents antibody drug conjugate species with 4 drug molecules conjugated to a single antibody molecule, peak 4 represents a mixture of antibody drug conjugate species, peak 5 represents antibody drug conjugate species with 6 drug molecules conjugated to a single antibody molecule. The identified antibody drug conjugate species differ by their average hydrophobicity, the one being more hydrophilic (e.g. left side) to hydrophobic (e.g. right side).

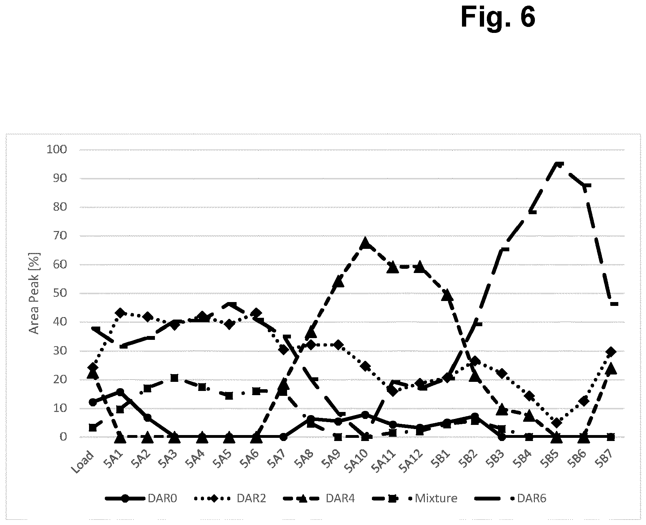

The analytical evaluation using hydrophobic interaction chromatography of collected fractions is displayed in the Figure 6, showing that the three obtained peaks are the separation of DAR species, namely the first peak is mainly the DAR2 and DAR6 species (e.g. elution maximum at 98 ml), second peak is mainly DAR4 species (e.g. elution maximum at 104 ml), and the third one - DAR 6 species (e.g. elution maximum at 125 ml).

As shown in Figure 6 while using a linear pH gradient elution the separation/enrichment of the different DAR's is possible. In the first peak of Figure 1 the purity of DAR2 is increased up to 40% (start level DAR2 24%). In the second peak the purity of DAR4 is increased up to 65% (start level 22%). In the third peak the purity of DAR6 is increased up to 95% (start level 37%).

### 3.

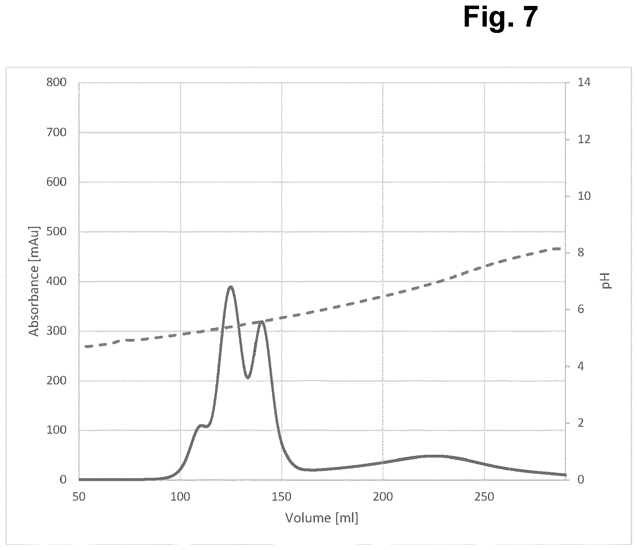

In this experiment, an ion exchange material prepared according to Example 1 (e.g. with the average particle size between 20-63 µm, the average pore size between 40-110 nm and an ionic density between 400 - 900 µeq/g) was evaluated for its ability to separate ADC species of commercially available drug Adcetris^{®}. The ion exchange material was packed in a chromatographic column of 5 × 100 mm dimensions with asymmetry between 0,8 - 1,2 and >3000 plates/m. After packing the ion exchange material, the obtained chromatographic column was cleaned with 1 M NaOH solution for 30 minutes and preequilibrated with loading buffer solution having pH of 4.5 and 300 mM NaCl (e.g. 24 mS/cm). The buffer solution contained combination of salts such as sodium dihydrogen phosphate, TRIS, glycine to obtain the pH of 4.5. The same solution was used to dissolve the lyophilized Adcetris^{®} sample till 1 mg/ml concentration. This solution was loaded on the prepared chromatographic column till 30 mg Adcetris^{®} loading was reached for 1 ml resin. These steps and the following steps were performed@ 150 cm/h buffer velocity. After loading 30 mg/ml Adcetris^{®}, the chromatographic column was washed with pH 4.5 buffer solution and then eluted used gradient elution with buffer having pH of 8.5 and 300 mM NaCl. This elution buffer was prepared using different salts such as sodium dihydrogen phosphate, TRIS, glycine. The conductivity and pH values were traced during the experimental set-up, showing that the Adcetris^{®} elution from the column was achieved due to the pH change during the gradient elution. The sample elution was fractionated and obtained fractions were evaluated of line using two different methods for the DAR species identification. The first method of choice was analytical hydrophobic interaction chromatography using mAbPac HIC-Butyl column with 4.6 × 100mm dimensions from Thermo Fisher Scientific. The chromatographic separation of fractionated samples enabled us to identify different DAR species and the quality thereof based on different elution window for each specie (e.g. the elution of DAR0 species was between 7-9 minute, the elution of DAR2 species between 10-14 minute, the elution of DAR4 species between 15-18 minute and the elution of DAR6 species between 19-22 minute). The second method of choice was Mass Spectroscopy, where different DAR species are separated based on their mass and ionization ratio. Using this method qualitative sample evaluation is possible, but not the quantitative, due to the different ionization of the DAR species (e.g. higher DAR species tend to ionize to lower degree). The example chromatographic experiment of 30 mg/ml Adcetris^{®} loading is shown in Figure 7, where UV adsorption signal trace in solid line and pH signal trace in dashed line.

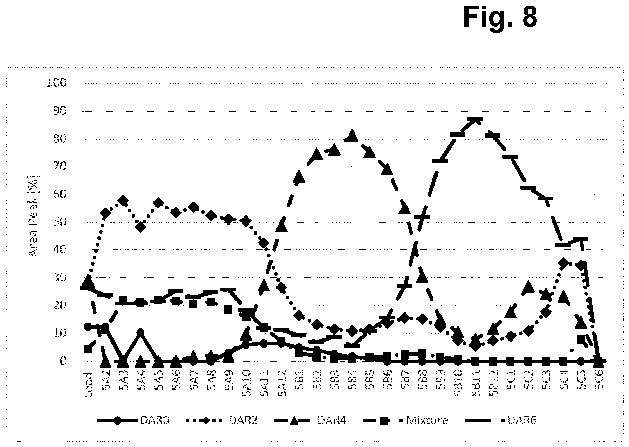

The analytical evaluation using hydrophobic interaction chromatography of collected fractions is displayed in the Figure 8, showing that the three obtained peaks are the separation of DAR species, namely the first peak is mainly the DAR2 species (e.g. elution maximum@ 110 ml), second peak is mainly DAR4 species (e.g. elution maximum@125 ml), and the third one - DAR6 species (e.g. elution maximum@140 ml).

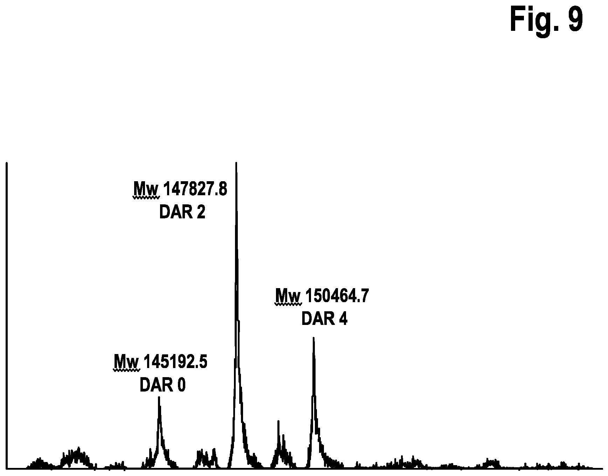

As shown in Figure 8 while using a linear pH gradient elution the separation/enrichment of the different DAR's is possible. In the first peak of Figure 7 the purity of DAR2 is increased up to 55% (start level 28%). In the second peak the purity of DAR4 is increased up to 81% (start level 29%). In the third peak the purity of DAR6 is increased up to 87% (start level 26%).Selected sample fractions were additionally evaluated using mass spectrometry to evaluate the quality of the DAR species separation. The results shown in Figure 9-12 show, that each obtained fraction contained the identified DAR species.

Figure 9 shows the deconvoluted mass spectrum for the Adcetris^{®} sample, showing a distribution of DAR species raging from the DAR0 to DAR4 species. DAR6 species are not detectable due to low ionization and method overload of lower DAR species.

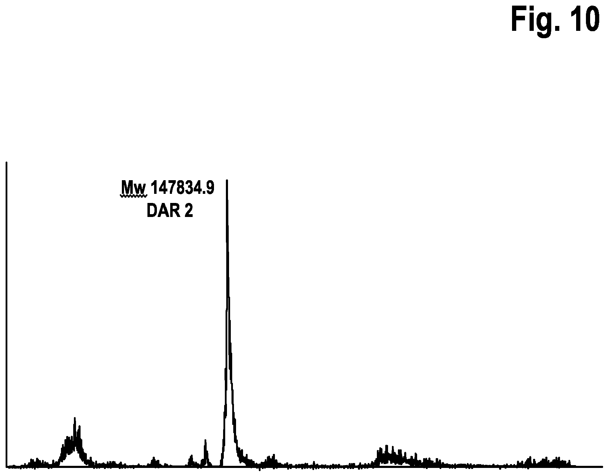

Figure 10 shows the deconvoluted mass spectrum for the elution fraction 5A7, representing the maximum of the first elution peak. The obtained molecular weight is for the DAR2 species, without obvious presence of other DAR species.

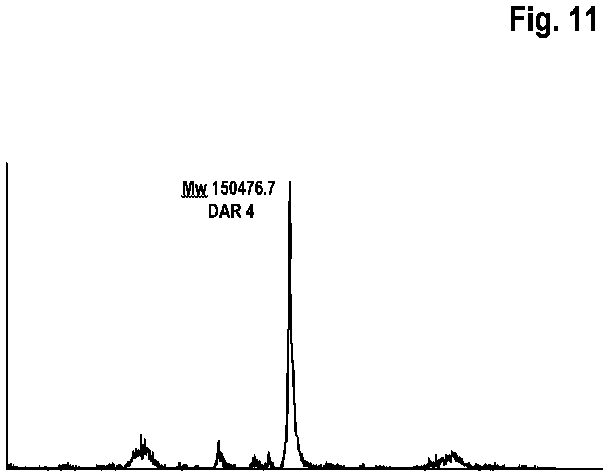

Figure 11 shows the deconvoluted mass spectrum for the elution fraction 5B4, representing the maximum of the second elution peak. The obtained molecular weight is for the DAR4 species, without obvious presence of other DAR species.

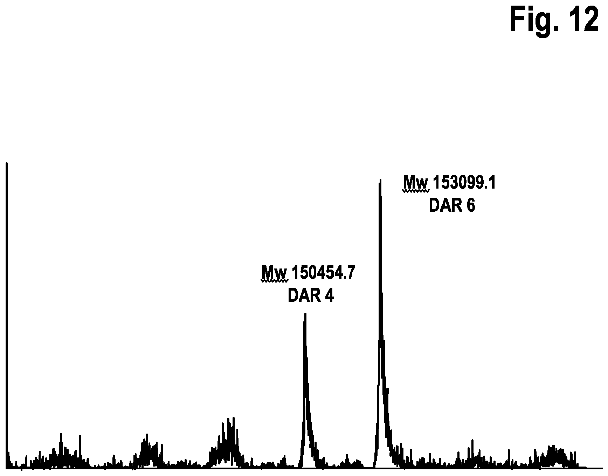

Figure 12 shows the deconvoluted mass spectrum for the elution fraction 5B11, representing the maximum of the third elution peak. The obtained molecular weight is for the DAR4 species and DAR6 species. The signal strength is not quantitative.

### 4.

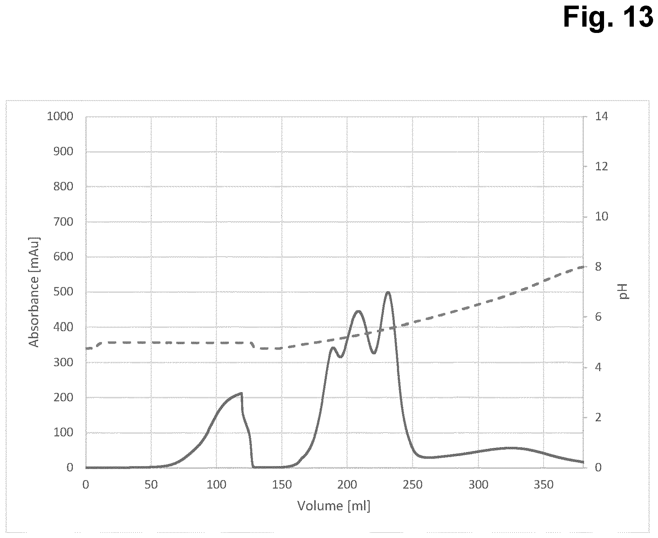

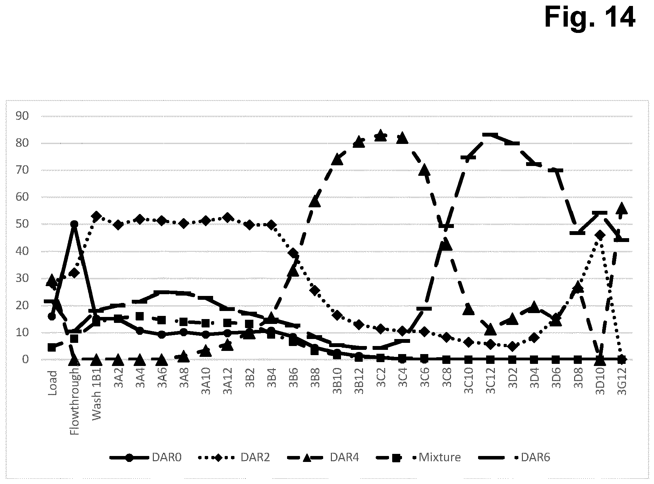

In this experiment, the ion exchange material prepared according to Example 1 (e.g. with the average particle size between 20-63 µm, the average pore size between 40-110 nm and an ionic density between 400 - 900 µeq/g) was evaluated for its ability to separate ADC species of commercially available drug Adcetris^{®}. The ion exchange material was packed in a chromatographic column of 5 × 100 mm dimensions with asymmetry between 0,8 - 1,2 and >3000 plates/m. After packing the ion exchange material, the obtained chromatographic column was cleaned with 1 M NaOH solution for 30 minutes and preequilibrated with loading buffer solution having pH of 4.5 and 300 mM NaCl (e.g. 24 mS/cm). The buffer solution contained a combination of salts such as sodium dihydrogen phosphate, TRIS, glycine to obtain the pH of 4.5. The same solution was used to dissolve the lyophilized Adcetris^{®} sample till 1 mg/ml concentration. This solution was loaded on the prepared chromatographic column till 60 mg Adcetris^{®} loading was reached for 1 ml resin. These steps and the following steps were performed@ 150 cm/h buffer velocity. After loading 60 mg/ml Adcetris^{®}, the chromatographic column was washed with pH 4.5 buffer solution and then eluted used gradient elution with buffer having pH of 8.5 and 300 mM NaCl. This elution buffer was prepared using different salts such as sodium dihydrogen phosphate, TRIS, glycine. The conductivity and pH values were traced during the experimental set-up, showing that the Adcetris^{®} elution from the column was achieved due to the pH change during the gradient elution. The sample elution was fractionated and obtained fractions were evaluated of line using two different methods for the DAR species identification. The first method of choice was analytical hydrophobic interaction chromatography using mAbPac HIC-Butyl column with 4.6 × 100mm dimensions from Thermo Fisher Scientific. The chromatographic separation of fractionated samples enabled us to identify different DAR species and the quality thereof based on different elution window for each specie (e.g. the elution of DAR0 species was between 7-9 minute, the elution of DAR2 species between 10-14 minute, the elution of DAR4 species between 15-18 minute and the elution of DAR6 species between 19-22 minute). The example chromatographic experiment of 60 mg/ml Adcetris^{®} loading is shown in Figure 13, where UV adsorption signal trace is represented in a solid line and pH signal trace in dashed line. The analytical evaluation using hydrophobic interaction chromatography of collected fractions is displayed in the Figure 14, showing that the three obtained peaks are the separation of DAR species, namely the first peak is mainly the DAR2 species (e.g. elution maximum@ 188 ml), second peak is mainly DAR4 species (e.g. elution maximum@205 ml), and the third one - DAR6 species (e.g. elution maximum@230 ml).

Surprisingly, also in 60 mg/ml loading, a separation of DAR species is possible, obtaining purified DAR2 species as seen in fraction 3B4 containing 49,66% of DAR 2 species. Moreover, the fraction 3C2 mainly contains DAR4 species with 83,02% and fraction 3C12 mainly contains DAR6 species with 83,12%. This result was obtained within the recovery rate of 97,37% accounting all chromatographic steps.

### 5.

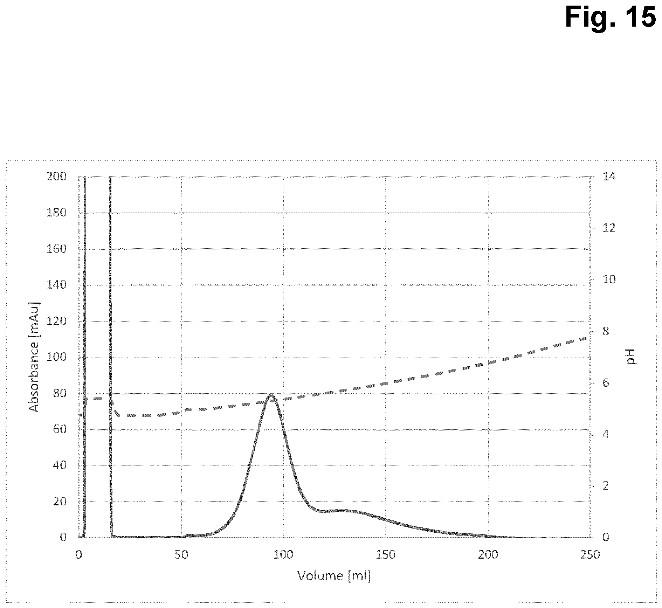

In this experiment, commercially available Capto^{™} MMC Impres chromatographic resin was evaluated for its ability to separate ADC species of commercially available drug Adcetris^{®}. This mixed mode material also bares weak cation exchange groups and hydrophobic groups, and could be suitable for the ADC specie separation. The Capto^{™} MMC Impres material was packed in a chromatographic column of 5 × 100 mm dimensions with asymmetry between 0,8 - 1,2 and >3000 plates/m. After packing the Capto^{™} MMC Impres material, the obtained chromatographic column was cleaned with 1 M NaOH solution for 30 minutes and preequilibrated with loading buffer solution having pH of 4.5 and 300 mM NaCl (e.g. 24 mS/cm). The buffer solution contained combination of salts such as sodium dihydrogen phosphate, TRIS, glycine to obtain the pH of 4.5. The same solution was used to dissolve the lyophilized Adcetris^{®} sample till 1 mg/ml concentration. This solution was loaded on the prepared chromatographic column till 30 mg Adcetris^{®} loading was reached for 1 ml resin. These steps and the following steps were performed@ 150 cm/h buffer velocity. After loading 30 mg/ml Adcetris^{®}, the chromatographic column was washed with pH 4.5 buffer solution and then eluted used gradient elution with buffer having pH of 8.5 and 300 mM NaCl. This elution buffer was prepared using different salts such as sodium dihydrogen phosphate, TRIS, glycine. The conductivity and pH values were traced during the experimental set-up, showing that the Adcetris^{®} elution from the column was achieved due to the pH change during the gradient elution. The sample elution was fractionated and obtained fractions were evaluated of line using two different methods for the DAR species identification. The first method of choice was analytical hydrophobic interaction chromatography using mAbPac HIC-Butyl column with 4.6 × 100mm dimensions from Thermo Fisher Scientific. The chromatographic separation of fractionated samples enabled us to identify different DAR species and the quality thereof based on different elution window for each specie (e.g. the elution of DAR0 species was between 7-9 minute, the elution of DAR2 species between 10-14 minute, the elution of DAR4 species between 15-18 minute and the elution of DAR6 species between 19-22 minute). The example chromatographic experiment of 30 mg/ml Adcetris^{®} loading is shown in Figure 15, where the UV adsorption signal trace is represented in a solid line and pH signal trace in dashed line.

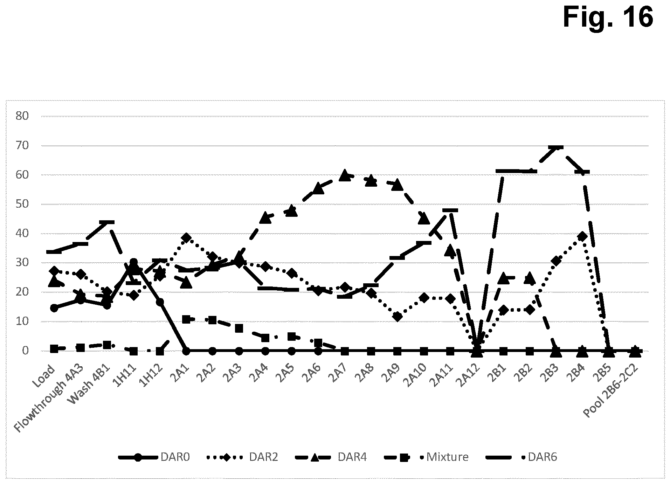

The analytical evaluation using hydrophobic interaction chromatography of collected fractions is displayed in the Figure 16, showing that the selected Capto^{™} MMC Impres resin was not able to bind all 30 mg of the ADC sample and that the separation was not so efficient. The maximum obtained purities for individual DAR species were ranging to 52,92% for DAR2, 75,32% for DAR4 and 72,74% for DAR6. As comparison, at same conditions the obtained purities on the innovative ion exchange resin were: DAR2 - 55.27%, DAR4 - 81,24% and DAR6 - 86,89%. Additionally, the recovery rates using the Capto MMC Impres resin were 88,38%.

### 6.

In this experiment the separation matrix is tested for its ability to separate different DAR (drug antibody ratio) of the ADC Adcetris^{®}. For the following example the resin was packed in a 5 mm diameter 100 mm long column using 20% ethanol 150 mM NaCl solution.

The two following buffers were prepared, for buffer A, being the load buffer, (e.g. 0.00796 M citric acid, 0.009068 M sodium di-hydrogen phosphate, 0.021543 M glycine, 0.010668 M TRIS, 0.007666 M succinate, 0.0111 M NaOH and 0.0889 M NaCl of pH 4.75) and the B buffer (e.g. 0.00796 M citric acid, 0.009068 M sodium di-hydrogen phosphate, 0.021543 M glycine, 0.010668 M TRIS, 0.007666 M succinate, 0.046 M NaOH and 0.054 M NaCl of pH 8.5).

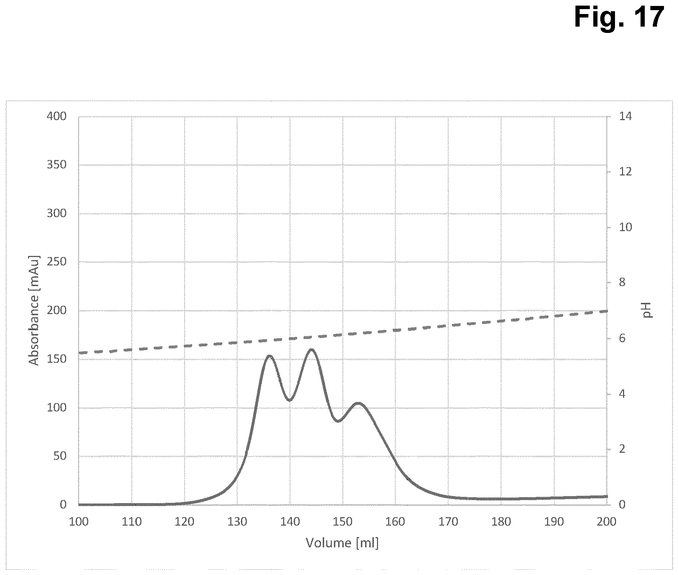

The packed column was equilibrated using buffer A for at least 10 column volumes at 0.491 ml/min. The lyophilized formulated ADC was dissolved in buffer A, to a concentration of 5 mg/ml. 3.93 ml of obtained solution was directly loaded on the equilibrated column at 0.491 ml/min. After loading, column was washed with 10 CV using equilibration solution. The elution of the bound ADC was performed using a pH gradient elution from 0-100% of buffer B in 120 CV at 0.491 ml/min (Figure 17).

**Table 1. Analytical results of sample fraction characterization displayed in quantitative area of elution fractions (peak 1-3), including loaded ADC (load) at 10 mg ADC/ ml CV loading and 50 mM NaCl**

| Sample | DAR0 % | DAR2 % | DAR4 % | Mixture % | DAR6 % |
|---|---|---|---|---|---|
| Load | 11.64 | 31.71 | 33.86 | 1.68 | 21.11 |
| Peak1 | 24.77 | 54.04 | 3.64 | 6.18 | 11.37 |
| Peak2 | 5.09 | 14.66 | 71.97 | 1.61 | 6.67 |
| Peak3 | 6.63 | 14.93 | 10.47 | 0.00 | 67.96 |

### 7.

In In this experiment the separation matrix is tested for its ability to separate different DAR (drug antibody ratio) of the ADC Adcetris^{®}. For the following example the resin was packed in a 5 mm diameter 100 mm long column using 20% ethanol 150 mM NaCl solution.

The two following buffers were prepared, for buffer A, being the load buffer, (e.g. 0.00796 M citric acid, 0.009068 M sodium di-hydrogen phosphate, 0.021543 M glycine, 0.010668 M TRIS, 0.007666 M succinate, 0.0111 M NaOH and 0.1389 M NaCl of pH 4.75) and the B buffer (e.g. 0.00796 M citric acid, 0.009068 M sodium di-hydrogen phosphate, 0.021543 M glycine, 0.010668 M TRIS, 0.007666 M succinate, 0.046 M NaOH and 0.104 M NaCl of pH 8.5).

The packed column was equilibrated using buffer A for at least 10 column

| Sample | DAR0 % | DAR2 % | DAR4 % | Mixture % | DAR6 % |
|---|---|---|---|---|---|
| Load | 11.64 | 31.71 | 33.86 | 1.68 | 21.11 |
| Peak1 | 17.49 | 62.31 | 0.00 | 7.65 | 12.55 |
| Peak2 | 2.48 | 8.90 | 82.32 | 0.00 | 6.30 |
| Peak3 | 6.62 | 13.30 | 10.72 | 0.00 | 69.35 |

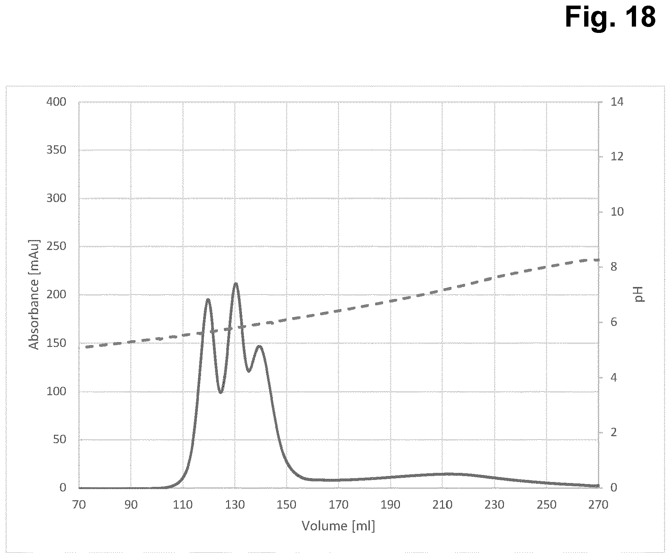

volumes at 0.491 ml/min. The lyophilized formulated ADC was dissolved in buffer A, to a concentration of 5 mg/ml. 3.93 ml of obtained solution was directly loaded on the equilibrated column at 0.491 ml/min. After loading, column was washed with 10 CV using equilibration solution. The elution of the bound ADC was performed using a pH gradient elution from 0-100% of buffer B in 120 CV at 0.491 ml/min (Figure 18).

Table 2. Analytical results of sample fraction characterization displayed in quantitative area of elution fractions (peak 1-3), including loaded ADC (load) at 10 mg ADC/ ml CV loading and 100 mM NaCl

### 8.

In this experiment the separation matrix is tested for its ability to separate different DAR (drug antibody ratio) of the ADC Adcetris^{®}. For the following example the resin was packed in a 5 mm diameter 100 mm long column using 20% ethanol 150 mM NaCl solution.

The two following buffers were prepared, for buffer A, being the load buffer, (e.g. 0.00796 M citric acid, 0.009068 M sodium di-hydrogen phosphate, 0.021543 M glycine, 0.010668 M TRIS, 0.007666 M succinate, 0.0111 M NaOH and 0.1889 M NaCl of pH 4.75) and the B buffer (e.g. 0.00796 M citric acid, 0.009068 M sodium di-hydrogen phosphate, 0.021543 M glycine, 0.010668 M TRIS, 0.007666 M succinate, 0.046 M NaOH and 0.154 M NaCl of pH 8.5).

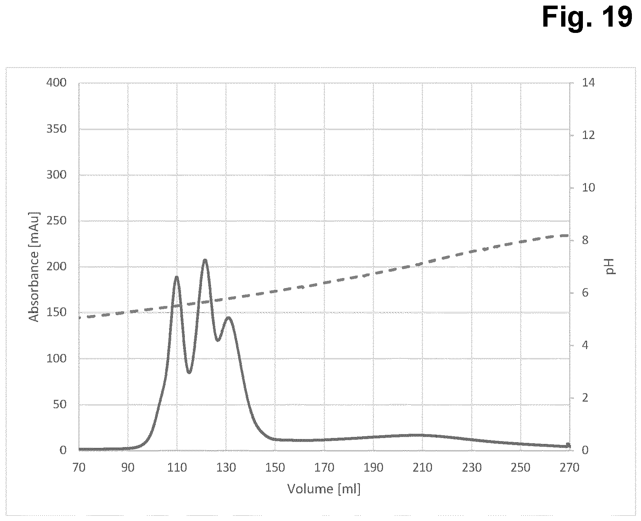

The packed column was equilibrated using buffer A for at least 10 column volumes at 0.491 ml/min. The lyophilized formulated ADC was dissolved in buffer A, to a concentration of 5 mg/ml. 3.93 ml of obtained solution was directly loaded on the equilibrated column at 0.491 ml/min. After loading, column was washed with 10 CV using equilibration solution. The elution of the bound ADC was performed using a pH gradient elution from 0-100% of buffer B in 120 CV at 0.491 ml/min (Figure 19).

**Table 3. Analytical results of sample fraction characterization displayed in quantitative area of elution fractions (peak 1-3), including loaded ADC (load) at 10 mg ADC/ ml CV loading and 150 mM NaCl**

| Sample | DAR0 % | DAR2 % | DAR4 % | Mixture % | DAR6 % |
|---|---|---|---|---|---|
| Peak1 | 8.64 | 66.49 | 0.00 | 9.62 | 15.26 |
| Peak2 | 2.31 | 10.26 | 87.42 | 0.00 | 0.00 |
| Peak3 | 2.61 | 14.22 | 11.40 | 0.00 | 71.76 |

### 9.

In this experiment the separation matrix is tested for its ability to separate different DAR (drug antibody ratio) of the ADC Adcetris^{®}. For the following example the resin was packed in a 5 mm diameter 100 mm long column using 20% ethanol 150 mM NaCl solution.

The two following buffers were prepared, for buffer A, being the load buffer, (e.g. 0.00796 M citric acid, 0.009068 M sodium di-hydrogen phosphate, 0.021543 M glycine, 0.010668 M TRIS, 0.007666 M succinate, 0.0111 M NaOH and 0.2389 M NaCl of pH 4.75) and the B buffer (e.g. 0.00796 M citric acid, 0.009068 M sodium di-hydrogen phosphate, 0.021543 M glycine, 0.010668 M TRIS, 0.007666 M succinate, 0.046 M NaOH and 0.204 M NaCl of pH 8.5).

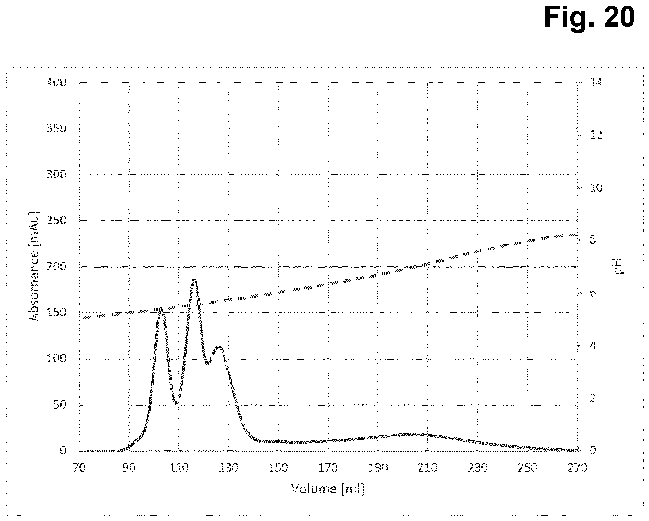

The packed column was equilibrated using buffer A for at least 10 column volumes at 0.491 ml/min. The lyophilized formulated ADC was dissolved in buffer A, to a concentration of 5 mg/ml. 3.93 ml of obtained solution was directly loaded on the equilibrated column at 0.491 ml/min. After loading, column was washed with 10 CV using equilibration solution. The elution of the bound ADC was performed using a pH gradient elution from 0-100% of buffer B in 120 CV at 0.491 ml/min (Figure 20).

**Table 4. Analytical results of sample fraction characterization displayed in quantitative area of elution fractions (peak 1-3), including loaded ADC (load) at 10 mg ADC/ ml CV loading and 200 mM NaCl.**

| Sample | DAR0 % | DAR2 % | DAR4 % | Mixture % | DAR6 % |
|---|---|---|---|---|---|
| Load | 11.64 | 31.71 | 33.86 | 1.68 | 21.11 |
| Peak1 | 2.62 | 68.60 | 0.00 | 9.81 | 18.96 |
| Peak2 | 2.41 | 9.95 | 87.64 | 0.00 | 0.00 |
| Peak3 | 0.00 | 16.17 | 7.73 | 0.00 | 76.10 |

### 10.

In this experiment the separation matrix is tested for its ability to separate different DAR (drug antibody ratio) of the ADC Adcetris^{®}. For the following example the resin was packed in a 5 mm diameter 100 mm long column using 20% ethanol 150 mM NaCl solution.

The two following buffers were prepared, for buffer A, being the load buffer, (e.g. 0.00796 M citric acid, 0.009068 M sodium di-hydrogen phosphate, 0.021543 M glycine, 0.010668 M TRIS, 0.007666 M succinate, 0.0111 M NaOH and 0.3389 M NaCl of pH 4.75) and the B buffer (e.g. 0.00796 M citric acid, 0.009068 M sodium di-hydrogen phosphate, 0.021543 M glycine, 0.010668 M TRIS, 0.007666 M succinate, 0.046 M NaOH and 0.304 M NaCl of pH 8.5).

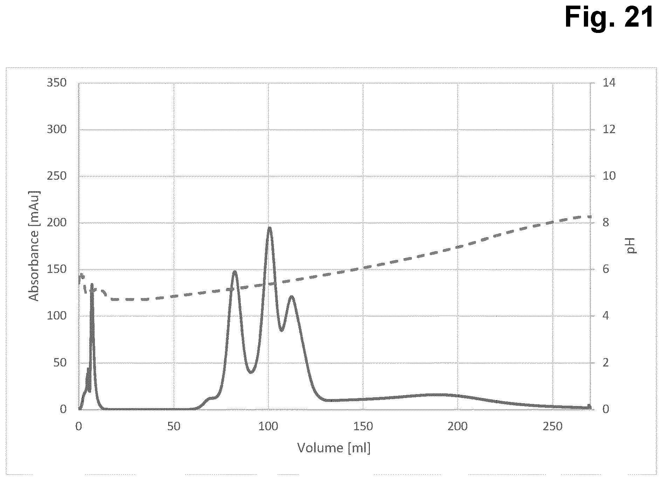

The packed column was equilibrated using buffer A for at least 10 column volumes at 0.491 ml/min. The lyophilized formulated ADC was dissolved in buffer A, to a concentration of 5 mg/ml. 3.93 ml of obtained solution was directly loaded on the equilibrated column at 0.491 ml/min. After loading, column was washed with 10 CV using equilibration solution. The elution of the bound ADC was performed using a pH gradient elution from 0-100% of buffer B in 120 CV at 0.491 ml/min (Figure 21).

**Table 5. Analytical results of sample fraction characterization displayed in quantitative area of elution fractions (peak 1-3), including loaded ADC (load) at 10 mg ADC/ ml CV loading and 300 mM NaCl.**

| Sample | DAR0 % | DAR2 % | DAR4 % | Mixture % | DAR6 % |
|---|---|---|---|---|---|
| Load | 11.64 | 31.71 | 33.86 | 1.68 | 21.11 |
| Flow through | 100.00 | 0.00 | 0.00 | 0.00 | 0.00 |
| Peak1 | 100.00 | 0.00 | 0.00 | 0.00 | 0.00 |
| Peak2 | 2.06 | 77.11 | 0.00 | 9.72 | 11.12 |
| Peak3 | 2.01 | 7.01 | 90.98 | 0.00 | 0.00 |
| Peak4 | 0.00 | 0.00 | 10.40 | 0.00 | 89.60 |

### 11.

In this experiment the separation matrix is tested for its ability to separate different DAR (drug antibody ratio) of the ADC Adcetris^{®}. For the following example the resin was packed in a 5 mm diameter 100 mm long column using 20% ethanol 150mM NaCl solution.

The two following buffers were prepared, for buffer A, being the load buffer, (e.g. 0.00796 M citric acid, 0.009068 M sodium di-hydrogen phosphate, 0.021543 M glycine, 0.010668 M TRIS, 0.007666 M succinate, 0.0111 M NaOH and 0.3889 M NaCl of pH 4.75) and the B buffer (e.g. 0.00796 M citric acid, 0.009068 M sodium di-hydrogen phosphate, 0.021543 M glycine, 0.010668 M TRIS, 0.007666 M succinate, 0.046 M NaOH and 0.354 M NaCl of pH 8.5).

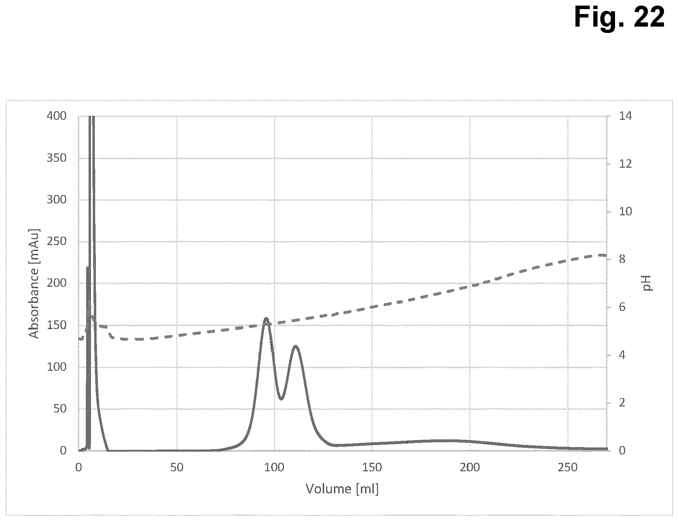

The packed column was equilibrated using buffer A for at least 10 column volumes at 0.491 ml/min. The lyophilized formulated ADC was dissolved in buffer A, to a concentration of 5 mg/ml. 3.93 ml of obtained solution was directly loaded on the equilibrated column at 0.491 ml/min. After loading, column was washed with 10 CV using equilibration solution. The elution of the bound ADC was performed using a pH gradient elution from 0-100% of buffer B in 120 CV at 0.491 ml/min (Figure 22).

**Table 6. Analytical results of sample fraction characterization displayed in quantitative area of elution fractions (peak 1-3), including loaded ADC (load) at 10 mg ADC/ ml CV loading and 350mM NaCl.**

| Sample | DAR0 % | DAR2 % | DAR4 % | Mixture % | DAR6 % |
|---|---|---|---|---|---|
| Load | 11.64 | 31.71 | 33.86 | 1.68 | 21.11 |
| Flow through | 27.74 | 58.51 | 0.00 | 6.50 | 7.26 |
| Peak1 | 0.00 | 9.18 | 90.82 | 0.00 | 0.00 |
| Peak2 | 0.00 | 0.00 | 8.59 | 0.00 | 91.41 |

### 12.

In this experiment the separation matrix is tested for its ability to separate different DAR (drug antibody ratio) of the ADC Adcetris^{®}. For the following example the resin was packed in a 5 mm diameter 100 mm long column using 20% ethanol 150 mM NaCl solution.

The two following buffers were prepared, for buffer A, being the load buffer, (e.g. 0.00796 M citric acid, 0.009068 M sodium di-hydrogen phosphate, 0.021543 M glycine, 0.010668 M TRIS, 0.007666 M succinate, 0.0111 M NaOH and 0.3389 M NaCl of pH 4.75) and the B buffer (e.g. 0.00796 M citric acid, 0.009068 M sodium di-hydrogen phosphate, 0.021543 M glycine, 0.010668 M TRIS, 0.007666 M succinate, 0.046 M NaOH and 0.304 M NaCl of pH 8.5).

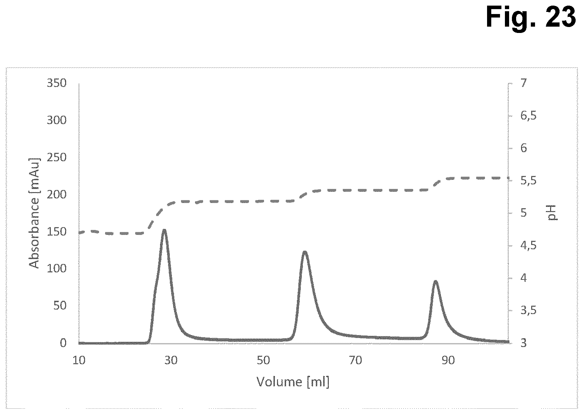

The packed column was equilibrated using buffer A for at least 10 column volumes at 0.491 ml/min. The lyophilized formulated ADC was dissolved in buffer A, to a concentration of 5 mg/ml. 3.93 ml of obtained solution was directly loaded on the equilibrated column at 0.491 ml/min. After loading, column was washed with 10 CV using equilibration solution. The elution of the bound ADC was performed using a pH steps at 22% B, 30% B and 36% B at 0.491 ml/min (Figure 23).

**Table 7. Analytical results of sample fraction characterization displayed in quantitative area of elution fractions (step 1-3), including loaded ADC (load) at 10 mg ADC/ ml CV loading and 300 mM NaCl in step modus.**

| Sample | DAR0 % | DAR2 % | DAR4 % | DAR6 % |
|---|---|---|---|---|
| Load | 11.89 | 26.34 | 28.23 | 31.25 |
| Step1 | | 54.14 | | |
| Step2 | | | 96.68 | |
| Step3 | | | | 93.26 |

### 13.

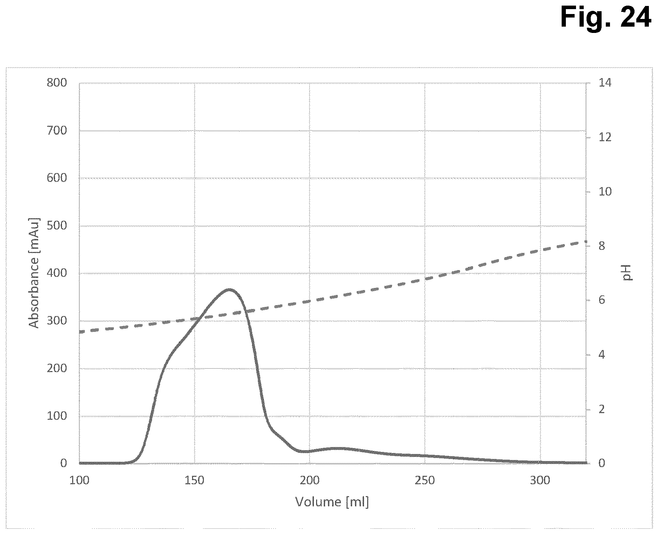

In this experiment, innovative ion exchange material was evaluated for its ability to separate ADC species of commercially available drug Kadcyla^{®}. The ion exchange material was packed in a chromatographic column of 5 x 100 mm dimensions with asymmetry between 0,8 - 1,2 and >3000 plates/m. After packing the ion exchange material, the obtained chromatographic column was cleaned with 1 M NaOH solution for 30 minutes and preequilibrated with loading buffer solution having pH of 4.5 and 300 mM NaCl (e.g. 24 mS/cm). The buffer solution contained combination of salts such as sodium dihydrogen phosphate, TRIS, glycine to obtain the pH of 4.5. The same solution was used to dissolve the lyophilized Kadcyla^{®} sample till 1 mg/ml concentration. This solution was loaded on the prepared chromatographic column till 30 mg Kadcyla^{®} loading was reached for 1 ml resin. These steps and the following steps were performed@ 150 cm/h buffer velocity. After loading 30 mg/ml Kadcyla^{®}, the chromatographic column was washed with pH 4.5 buffer solution and then eluted used gradient elution with buffer having pH of 8.5 and 300 mM NaCl. This elution buffer was prepared using different salts such as sodium dihydrogen phosphate, TRIS, glycine. The conductivity and pH values were traced during the experimental set-up, showing that the Kadcyla^{®} elution from the column was achieved due to the pH change during the gradient elution. The sample elution was fractionated and obtained fractions were evaluated of line using two different methods for the DAR species identification. The first method of choice was analytical hydrophobic interaction chromatography using mAbPac HIC-Butyl column with 4.6 x 100mm dimensions from Thermo Fisher Scientific. The chromatographic separation of fractionated samples enabled us to identify different DAR species and the quality thereof based on different elution window for each specie. The second method of choice was Mass Spectroscopy, where different DAR species are separated based on their mass and ionization ratio. Using this method qualitative sample evaluation is possible, but not the quantitative, due to the different ionization of the DAR species (e.g. higher DAR species tend to ionize to lower degree). The example chromatographic experiment of 30 mg/ml Kadcyla^{®} loading is shown in Figure 24, where the UV adsorption signal trace is represented in solid line and pH signal trace in dashed line.

The fractions were collected and were analyzed using analytical HIC (hydrophobic interaction chromatography) HPLC and the amount of different DARs calculated using the quantitative area. Figure 25 illustrates the separation of antibody drug conjugate species during a linear elution gradient on an analytical HPLC column. Five consecutive elution pools are identified in the UV signal trace, representing five different antibody drug conjugate species: peak 1 represents antibody drug conjugate species without drug, peak 2 represents antibody drug conjugate species with 1 drug molecule conjugated to a single antibody molecule, peak 3 represents antibody drug conjugate species with 2 drug molecules conjugated to a single antibody molecule, peak 4 represents antibody drug conjugate species with 3 drug molecules conjugated to a single antibody molecule, peak 5 represents antibody drug conjugate species with >4 drug molecules conjugated to a single antibody molecule. The identified antibody drug conjugate species differ by their average hydrophobicity, the one being more hydrophilic (e.g. left side) to hydrophobic (e.g. right side).

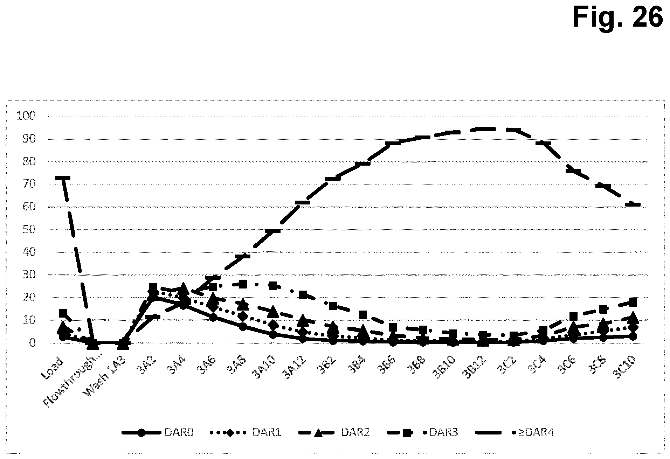

The analytical evaluation using hydrophobic interaction chromatography of collected fractions is displayed in the Figure 26, showing that the selected innovative ion exchange resin was able separate lower DAR species from the higher ones.

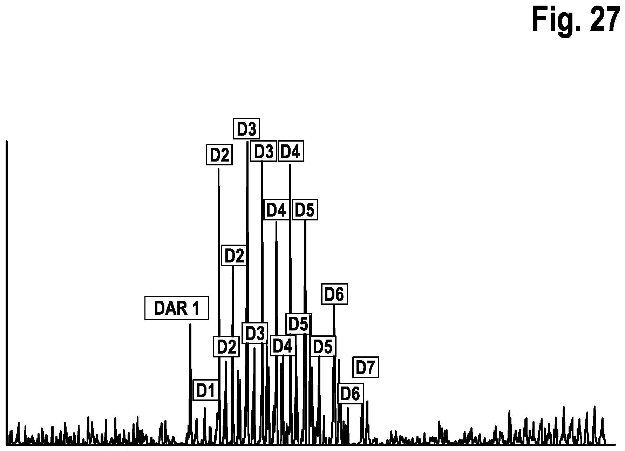

Selected sample fractions were additionally evaluated using mass spectrometry to evaluate the quality of the DAR specie separation. The results shown in figure 27-29 show, that each obtained fraction contained the identified DAR specie.

Figure 27. Deconvoluted mass spectrum for the Kadcyla^{®} sample, showing a distribution of DAR species raging from the DAR0 to DAR7 species.

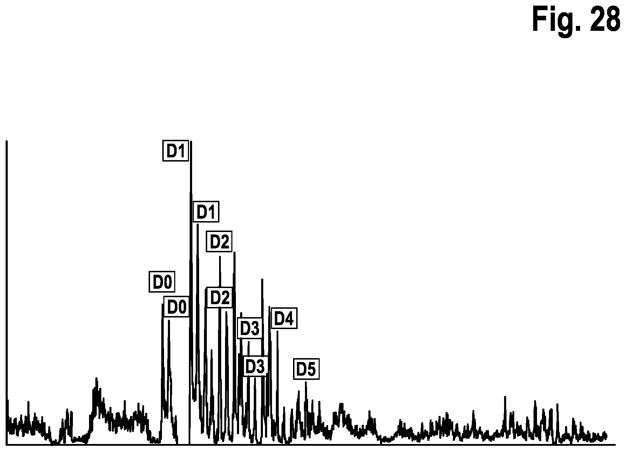

Figure 28. Deconvoluted mass spectrum for the elution fraction 3A4 sample, showing a distribution of lower DAR species.

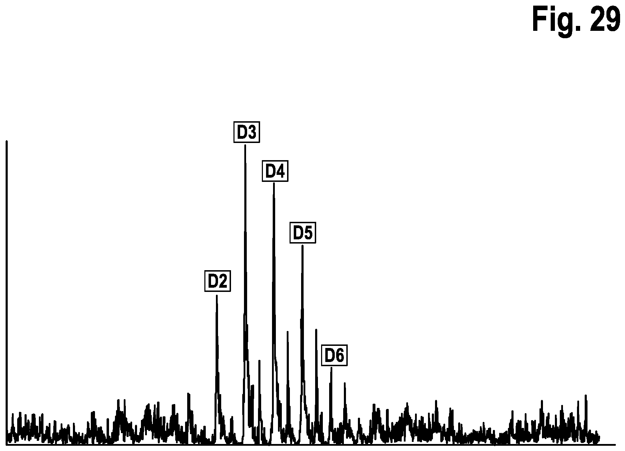

Figure 29. Deconvoluted mass spectrum for the elution fraction 3B12 sample, showing a distribution of higher DAR species.

### 14.

In this experiment the separation matrix is tested for its ability to separate different DAR (drug antibody ratio) of the ADC Kadcyla^{®}. For the following example the resin was packed in a 5 mm diameter 100 mm long column using 20% ethanol 150 mM NaCl solution.

The two following buffers were prepared, for buffer A, being the load buffer, (e.g. 0.00796 M citric acid, 0.009068 M sodium di-hydrogen phosphate, 0.021543 M glycine, 0.010668 M TRIS, 0.007666 M succinate, 0.0111 M NaOH and 0.2889 M NaCl of pH 4.75 and conductivity of -28 mS/cm) and the B buffer (e.g. 0.00796 M citric acid, 0.009068 M sodium di-hydrogen phosphate, 0.021543 M glycine, 0.010668 M TRIS, 0.007666 M succinate, 0.046 M NaOH and 0.254 M NaCl of pH 8.5 and conductivity of -27 mS/cm).

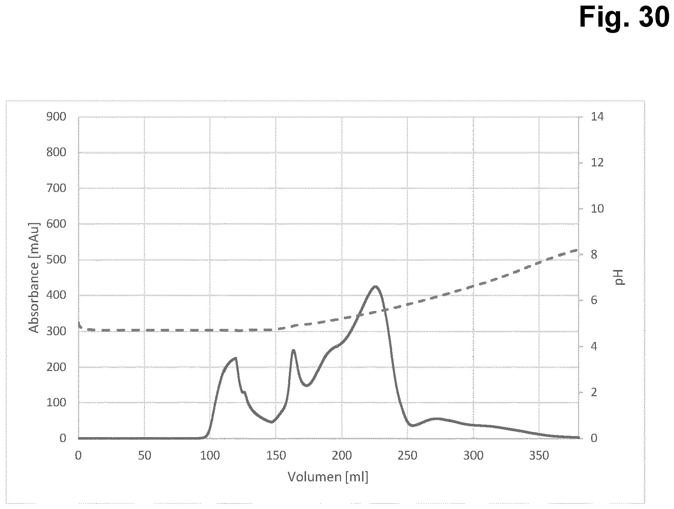

The packed column was equilibrated using buffer A for at least 10 column volumes at 0.491 ml/min. The lyophilized formulated ADC was dissolved in buffer A, to a concentration of 5 mg/ml. 22.52 ml of obtained solution was directly loaded on the equilibrated column at 0.491 ml/min. After loading, column was washed with 10 CV using equilibration solution. The elution of the bound ADC was performed using a pH gradient elution from 0-100% of buffer B in 120 CV at 0.491 ml/min (Figure 30).

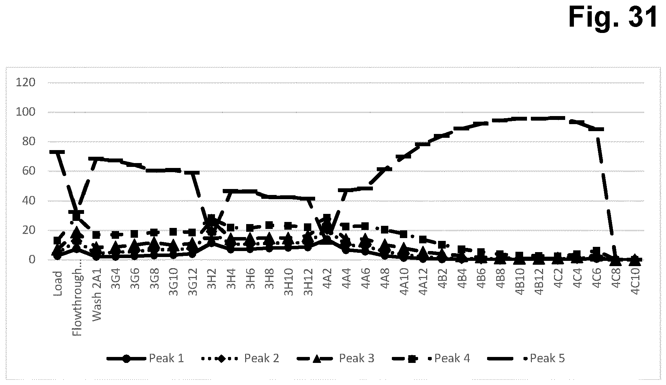

As shown in Figure 31, using a linear pH gradient elution, the separation/enrichment of the different DAR's is possible. The purity of >DAR4 is increased up to 96% (start level 68%).

### 15.

In this experiment the Capto^{™} MMC ImpRes chromatography resin is tested for its ability to separate different DAR (drug antibody ratio) of the ADC Kadcyla^{®}. For the following example the resin was packed in a 5 mm diameter 100 mm long column using 20% ethanol 150 mM NaCl solution. The two following buffers were prepared, for buffer A, being the load buffer, (e.g. 0.00796 M citric acid, 0.009068 M sodium di-hydrogen phosphate, 0.021543 M glycine, 0.010668 M TRIS, 0.007666 M succinate, 0.0111 M NaOH and 0.2889 M NaCl of pH 4.75 and conductivity of -28 mS/cm) and the B buffer (e.g. 0.00796 M citric acid, 0.009068 M sodium di-hydrogen phosphate, 0.021543 M glycine, 0.010668 M TRIS, 0.007666 M succinate, 0.046 M NaOH and 0.254 M NaCl of pH 8.5 and conductivity of -27 mS/cm).

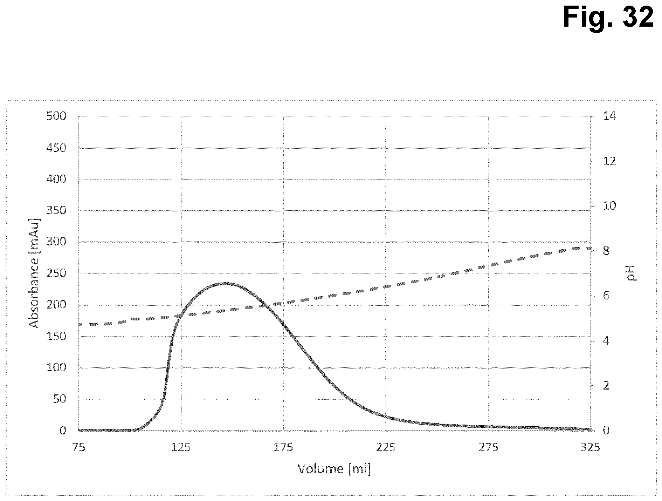

The packed column was equilibrated using buffer A for at least 10 column volumes at 0.491 ml/min. The lyophilized formulated ADC was dissolved in buffer A, to a concentration of 5 mg/ml. 11.26 ml of obtained solution was directly loaded on the equilibrated column at 0.491 ml/min. After loading, column was washed with 10 CV using equilibration solution. The elution of the bound ADC was performed using a pH gradient elution from 0-100% of buffer B in 120 CV at 0.491 ml/min (Figure 32).

As shown in Figure 32, using a linear pH gradient elution, the separation/enrichment of the different DAR's is possible. The purity of >DAR4 is increased up to >90% (start level 72%).

### 16.

In this experiment the separation matrix is tested for its ability to separate different DAR (drug antibody ratio) of the ADC Kadcyla^{®}. For the following example the resin was packed in a 5 mm diameter 100 mm long column using 20% ethanol 150 mM NaCl solution.

The two following buffers were prepared, for buffer A, being the load buffer, (e.g. 0.00796 M citric acid, 0.009068 M sodium di-hydrogen phosphate, 0.021543 M glycine, 0.010668 M TRIS, 0.007666 M succinate, 0.0111 M NaOH and 0.4389 M NaCl of pH 4.75) and the B buffer (e.g. 0.00796 M citric acid, 0.009068 M sodium di-hydrogen phosphate, 0.021543 M glycine, 0.010668 M TRIS, 0.007666 M succinate, 0.046 M NaOH and 0.404 M NaCl of pH 8.5).

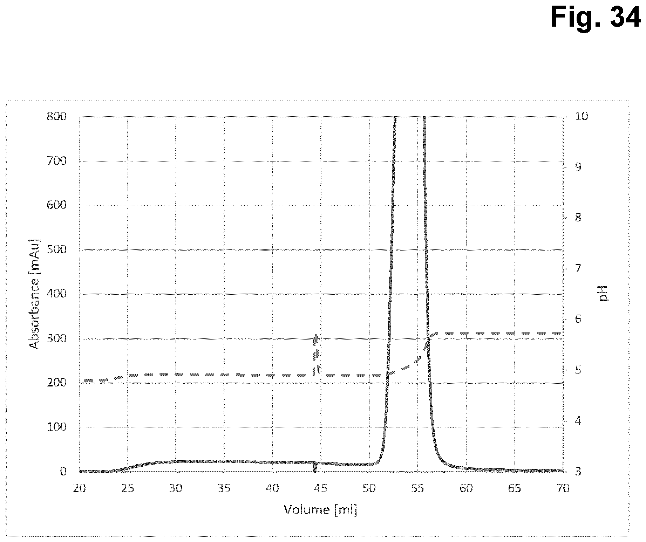

The packed column was equilibrated using buffer A for at least 10 column volumes at 0.491 ml/min. The lyophilized formulated ADC was dissolved in buffer A, to a concentration of 5 mg/ml. 3.93 ml of obtained solution was directly loaded on the equilibrated column at 0.491 ml/min. After loading, column was washed with 10 CV using equilibration solution. The elution of the bound ADC was performed using a pH steps at 5% B and 40% B at 0.491 ml/min (Figure 34).

**Table 8. Analytical results of sample fraction characterization displayed in quantitative area of elution fractions (step 1-2), including loaded ADC (load) at 10 mg ADC/ ml CV loading and 400 mM NaCl in step modus.**

| Sample | DAR0 % | DAR1 % | DAR2 % | DAR3 % | > DAR4 % |
|---|---|---|---|---|---|
| Load | 2.11 | 4.44 | 5.73 | 10.59 | 77.13 |
| Step1 | 18.36 | 22.46 | 25.10 | 34.08 | 0.00 |
| Step2 | 1.01 | 0.91 | 0.91 | 0.91 | 96.25 |

## Claims

1. A separation material comprising a hydroxyl-group containing base matrix, to the surfaces of which polymer chains are grafted by covalent bonding, **characterized in that** the polymer chains comprise amino acid end groups - N(Y)-R3 with
a. Y being independently from each other H or CH3, preferably H, and
b. R3 being -CHCOOMR4
with R4 being C1 to C4 alkyl or C1 to C4 perfluoroalkyl and M being H, Na, K, or NH₄.

2. Separation material according to claim1 whereby the monomer units of the polymer chains are linked in a linear manner and each monomer unit comprises an end group -N(Y)-R3.

3. Separation material according to one or more of claim1 to 2 whereby the ionic density is between 400 - 900µeq/g.

4. Separation material according to one or more of claim1 to 3 whereby Y is H and R4 is isopropyl and/or isobutyl.

5. Separation material according to one or more of claim1 to 4 whereby the hydroxylgroup containing base matrix comprises aliphatic hydroxyl groups.

6. Separation material according to one or more of claims 1 to 5 whereby the base matrix is formed by copolymerisation of a hydrophilically substituted alkyl vinyl ether selected from the group of 1,4-butanediol monovinyl ether, 1,5-pentanediol monovinyl ether, diethylene glycol monovinyl ether or cyclo¬hexane¬dimethanol monovinyl ether and divinylethyleneurea (1,3-divinylimidazolin-2-one) as crosslinking agent.

7. A process for the chromatographic purification and/or separation of antibody drug conjugates (ADCs) by contacting the sample comprising the ADCs with a separation material comprising a hydroxyl group containing base matrix, to the surfaces of which polymer chains are grafted by covalent bonding, **characterized in that** the polymers comprise end groups -N(Y)-R3 with Y being independently from each other H or CH3
R3 being -CHCOOMR4
with R4 being C1 to C4 alkyl or C1 to C4 perfluoroalkyl,
and M being H, Na, K, or NH₄.

8. Process according to claim 7 whereby the ADC target molecules are bound to the separation matrix at a pH between 2 and 7, optionally washed and eluted by pH change to alkaline pH.

9. Process according to claim 7 or 8 whereby the sample is applied to the separation matrix at an ionic density between 10-1200peq/g.

10. Process according to one or more of claims 7 to 9, whereby between 10 mg and 100 mg ADCs are bound per ml of the separation material.

11. Process for the preparation of a separation material according to one or more of claims 1 to 6 by subjecting the hydroxyl group containing base matrix to a cerium (IV) catalyzed graft polymerization of monomers according to formula V 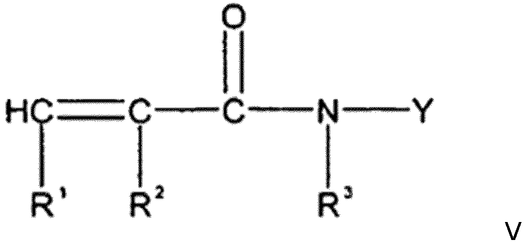
with R1, R2 and Y being independently from each other H or CH3,
R3 being -CHCOOMR4
with R4 being C1 to C4 alkyl or C1 to C4 perfluoroalkyl, and M being H, Na, K, or NH_{4.}

## Patentansprüche

1. Trennmaterial enthaltend eine hydroxygruppenhaltige Basismatrix, auf deren Oberflächen durch kovalente Bindung Polymerketten gepfropft sind, **dadurch gekennzeichnet, dass** die Polymerketten Aminosäure-Endgruppen -N(Y)-R3 enthalten, wobei
a. Y unabhängig voneinander H oder CH3, vorzugsweise H, ist, und
b. R3 -CHCOOMR4 ist,
wobei R4 C1- bis C4-Alkyl oder C1- bis C4-Perfluoralkyl ist und M H, Na, K oder NH₄ ist.

2. Trennmaterial nach Anspruch 1, wobei die Monomereinheiten der Polymerketten auf lineare Weise verknüpft sind und jede Monomereinheit eine Endgruppe -N(Y)-R3 enthält.

3. Trennmaterial nach einem oder mehreren von Anspruch 1 bis 2, wobei die Ionendichte zwischen 400 - 900 µÄq/g liegt.

4. Trennmaterial nach einem oder mehreren von Anspruch 1 bis 3, wobei Y H ist und R4 Isopropyl und/oder Isobutyl ist.

5. Trennmaterial nach einem oder mehreren von Anspruch 1 bis 4, wobei die hydroxygruppenhaltige Basismatrix aliphatische Hydroxygruppen enthält.

6. Trennmaterial nach einem oder mehreren der Ansprüche 1 bis 5, wobei die Basismatrix durch Copolymerisation eines aus der Gruppe 1,4-Butandiolmonovinyl-ether, 1,5-Pentandiol-monovinyl-ether, Diethylenglykol-monovinyl-ether oder Cyclohexan-dimethanol-monovinyl-ether ausgewählten hydrophil substituierten Alkyl-vinyl-ethers und Divinylethylen-harnstoff (1,3-Divinyl-imidazolin-2-on) als Vernetzungsmittel gebildet wird.

7. Verfahren zur chromatographischen Reinigung und/oder Trennung von Antikörper-Wirkstoff-Konjugaten (ADCs) durch In-Kontakt-Bringen der die ADCs enthaltenden Probe mit einem Trennmaterial enthaltend eine hydroxygruppenhaltige Basismatrix, auf deren Oberflächen durch kovalente Bindung Polymerketten gepfropft sind, **dadurch gekennzeichnet, dass** die Polymere Endgruppen -N(Y)-R3 enthalten, wobei
Y unabhängig voneinander H oder CH3 ist,
R3 -CHCOOMR4 ist,
wobei R4 C1- bis C4-Alkyl oder C1- bis C4-Perfluoralkyl ist,
und M H, Na, K oder NH₄ ist.

8. Verfahren nach Anspruch 7, wobei die ADC-Zielmoleküle bei einem pH zwischen 2 und 7 an die Trennmatrix gebunden, gegebenenfalls gewaschen und durch einen pH-Wechsel auf einen alkalischen pH eluiert werden.

9. Verfahren nach Anspruch 7 oder 8, wobei die Probe mit einer Ionendichte zwischen 10-1200 µÄq/g auf die Trennmatrix aufgebracht wird.

10. Verfahren nach einem oder mehreren der Ansprüche 7 bis 9, wobei pro ml des Trennmaterials zwischen 10 mg und 100 mg ADCs gebunden werden.

11. Verfahren zur Herstellung eines Trennmaterials nach einem oder mehreren der Ansprüche 1 bis 6, indem man die hydroxygruppenhaltige Basismatrix einer Cerium(IV)-katalysierten Pfropfpolymerisation von Monomeren gemäß der Formel V 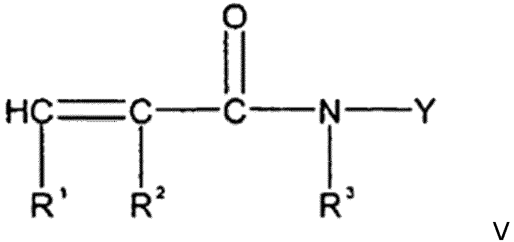
unterwirft, wobei R1, R2 und Y unabhängig voneinander H oder CH3 sind, R3 -CHCOOMR4 ist,
wobei R4 C1- bis C4-Alkyl oder C1- bis C4-Perfluoralkyl ist und M H, Na, K oder NH₄ ist.

## Revendications

1. Matériau de séparation comprenant une matrice de base à groupements hydroxyle, aux surfaces de laquelle des chaînes de polymère sont greffées par liaison covalente, **caractérisé en ce que** les chaînes de polymère comprennent des groupements terminaux de type acide aminé -N(Y)-R3,
a. Y étant indépendamment les uns des autres H ou CH₃, préférablement H, et
b. R3 étant -CHCOOMR4
R4 étant alkyle en C1 à C4 ou perfluoroalkyle en C1 à C4 et M étant H, Na, K, ou NH₄.

2. Matériau de séparation selon la revendication 1, dans lequel les motifs monomères des chaînes de polymère sont liés de manière linéaire et chaque motif monomère comprend un groupement terminal -N(Y)-R3.

3. Matériau de séparation selon l'une ou plusieurs parmi les revendications 1 à 2, dans lequel la densité ionique est de 400 - 900 µéq./g.

4. Matériau de séparation selon l'une ou plusieurs parmi les revendications 1 à 3, dans lequel Y est H et R4 est isopropyle et/ou isobutyle.

5. Matériau de séparation selon l'une ou plusieurs parmi les revendications 1 à 4, dans lequel la matrice de base à groupements hydroxyle comprend des groupements hydroxyle aliphatiques.

6. Matériau de séparation selon l'une ou plusieurs parmi les revendications 1 à 5, dans lequel la matrice de base est formée par copolymérisation d'un alkyl-vinyl éther à substitution hydrophile choisi dans le groupe constitué par l'éther monovinylique de 1,4-butanediol, l'éther monovinylique de 1,5-pentanediol, l'éther monovinylique de diéthylène glycol ou l'éther monovinylique de cyclohexanediméthanol et la divinyléthylèneurée (1,3-divinyl-imidazolin-2-one) comme agent de réticulation.

7. Procédé de purification et/ou séparation chromatographique de conjugués anticorps-médicaments (ADC) par la mise en contact de l'échantillon comprenant les ADC avec un matériau de séparation comprenant une matrice de base à groupements hydroxyle, aux surfaces de laquelle des chaînes de polymère sont greffées par liaison covalente, **caractérisé en ce que** les polymères comprennent des groupements terminaux -N(Y)-R3,
Y étant indépendamment les uns des autres H ou CH₃
R3 étant -CHCOOMR4,
R4 étant alkyle en C1 à C4 ou perfluoroalkyle en C1 à C4,
et M étant H, Na, K, ou NH₄.

8. Procédé selon la revendication 7, dans lequel les molécules cibles d'ADC sont liées à la matrice de séparation à un pH compris entre 2 et 7, éventuellement lavées et éluées par un changement de pH pour un pH alcalin.

9. Procédé selon la revendication 7 ou 8, dans lequel l'échantillon est appliqué à la matrice de séparation selon une densité ionique de 10-1200 µéq./g.

10. Procédé selon l'une ou plusieurs parmi les revendications 7 à 9, dans lequel entre 10 mg et 100 mg d'ADC sont liés par ml du matériau de séparation.

11. Procédé de préparation d'un matériau de séparation selon l'une ou plusieurs parmi les revendications 1 à 6, en soumettant la matrice de base à groupements hydroxyle à une polymérisation par greffage catalysée par du cérium (IV) de monomères répondant à la formule V 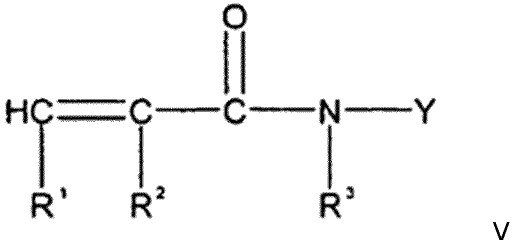
R1, R2 et Y étant indépendamment les uns des autres H ou CH₃,
R3 étant -CHCOOMR4,
R4 étant alkyle en C1 à C4 ou perfluoroalkyle en C1 à C4, et M étant H, Na, K, ou NH₄.
